(19) Europäisches Patentamt
European Patent Office
Office européen des brevets

(11) Veröffentlichungsnummer: **0 005 848**
**B1**

# (12) EUROPÄISCHE PATENTSCHRIFT

(45) Veröffentlichungstag der Patentschrift: **30.12.81**

(21) Anmeldenummer: **79101724.7**

(22) Anmeldetag: **01.06.79**

(51) Int. Cl.³: **C 07 C 103/26,** C 07 B 19/00, C 07 D 213/38, C 07 D 307/52, C 07 D 333/20, A 61 K 31/34, A 61 K 31/38, A 61 K 31/44 // A61K31/165, C07C69/84, C07C91/22, C07C101/42, C07C103/34, C07D265/22

(54) **N-Alkylierte Aminoalkohole und ihre Salze, Verfahren zu deren Herstellung und diese enthaltende pharmazeutische Präparate.**

(30) Priorität: **05.06.78 CH 6136/78**

(43) Veröffentlichungstag der Anmeldung: **12.12.79 Patentblatt 79/25**

(45) Bekanntmachung des Hinweises auf die Patenterteilung: **30.12.81 Patentblatt 81/52**

(84) Benannte Vertragsstaaten: **AT BE CH DE FR GB IT LU NL SE**

(56) Entgegenhaltungen:
**DE-A-1 543 538**
**DE-B-2 032 642**
**DE-A-2 135 678**
**DE-A-2 357 849**
**GB-A-1 178 400**
**US-A-4 082 772**

(73) Patentinhaber: **CIBA-GEIGY AG, Patentabteilung Postfach, CH-4002 Basel (CH)**

(72) Erfinder: **Ostermayer, Franz, Dr., Am Hang 5, CH-4125 Riehen (CH)**
Erfinder: **Zimmermann, Markus, Dr., Steinbrecheweg 8, 4125 Riehen (CH)**

(74) Vertreter: **Zumstein sen., Fritz, Dr. et al, Bräuhausstrasse 4, D-8000 München 2 (DE)**

**EP 0 005 848 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# N-Alkyliertes Aminoalkohole und ihre Salze, Verfahren zu deren Herstellung und diese enthaltende pharmazeutische Präparate

Die vorliegende Erfindung betrifft neue N-alkylierte Aminoalkohole der Formel

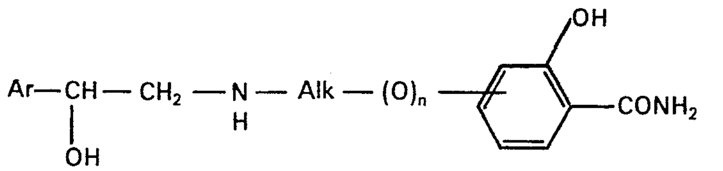

(I),

worin Ar einen unsubstituierten oder durch Hydroxy substituierten Rest aromatischen Charakters, n die Werte Null oder 1 und Alk einen Alkylenrest mit 2 bis 5 Kohlenstoffatomen bedeuten, wobei das Stickstoffatom und das Sauerstoffatom, oder, falls n für Null steht, der Phenylrest durch mindestens zwei Kohlenstoffatome voneinander getrennt sind, und deren Salze, Verfahren zu deren Herstellung sowie pharmazeutische Präparate enthaltend solche Verbindungen und deren Anwendung.

Aus der DE-OS 21 35 678 sind 1-Phenyl-2-alkylaminoalkane und α-Aminoalkylphenylketone und insbesondere 1-Phenyl-2-alkylamino-alkanole bekannt, von denen die letztgenannte Gruppe von Verbindungen bezüglich ihrer chemischen Struktur sich von den anmeldungsgemässen Verbindungen der Formel I im wesentlichen durch das Fehlen der in der Salicylamidgruppe stehenden aromatischen Hydroxygruppe unterscheidet. Die bekannten Stoffe besitzen die Eigenschaft, die β-adrenergischen Rezeptoren zu stimulieren. Insbesondere erhöhen sie die Kraft der Herzmuskelkontraktion und sind zur Heilbehandlung oder zur Prophylaxe von Herzkrankheiten, wie Herzversagen durch Blutandrang brauchbar.

Ein Rest aromatischen Charakters Ar ist in erster Linie ein monocyclischer carbo- oder heterocyclischer Rest aromatischen Charakters mit 5 bis 6 Ringgliedern und höchstens 3 Heteroatomen, in erster Linie Stickstoff-, Sauerstoff- und/oder Schwefelatomen, als Ringglieder. Ein Rest Ar ist daher insbesondere Phenyl, ferner Pyridyl, Pyridazinyl, Pyrimidinyl, Pyrazinyl, Furyl, Pyrryl, Thienyl, Oxazolyl, Thiazolyl, Pyrazolyl, Imidazolyl oder Thiadiazolyl.

Die Gruppe der Formel

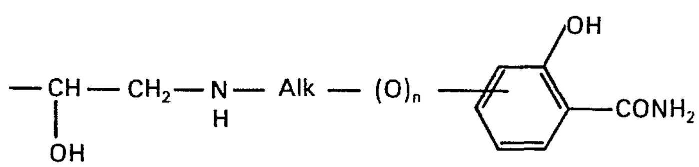

ist an ein Kohlenstoffatom in einem Rest Ar gebunden und kann irgendeine der möglichen Stellungen einnehmen. In einem durch eine Hydroxygruppe substituierten Phenylrest steht die genannte Gruppe vorzugsweise in 4-Stellung. Der Rest der Formel

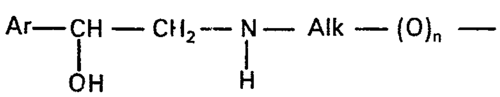

kann den Salicylamidring in jeder beliebigen der möglichen Stellungen substituieren.

Alkylen Alk kann geradkettig oder verzweigt sein und ist. z.B. 1,2-Aethylen, 1,2-, 2,3- oder 1,3-Propylen, 1,4- oder 2,4-Butylen oder 2-Methyl-2,4-Butylen, wobei vorzugsweise das mit dem Stickstoffatom verbundene Kohlenstoffatom verzweigt ist.

Pyridyl ist 2-, 3- oder 4-Pyridyl; Pyridazinyl ist 3- oder 4-Pyridazinyl; Pyrimidinyl ist 2-, 4- oder 5-Pyrimidinyl, während Pyrazinyl 2-Pyrazinyl darstellt. Furyl ist 2- oder 3-Furyl; Pyrryl ist 2- oder 3-Pyrryl; Thienyl ist 2- oder 3-Thienyl, während Oxazolyl 2- oder 4-Oxazolyl, Thiazolyl 2-, 4- oder 5-Thiazolyl, Pyrazolyl 3- oder 4-Pyrazolyl, Imidazolyl 2- oder 4-Imidazolyl und Thiadiazolyl 1,2,4-Thiadiazol- 3- oder -5-yl, oder 1,3,4-Thiadiazol-2-yl darstellen.

Salze von Verbindungen der Formel I sind in erster Linie Säureadditionssalze und insbesondere pharmazeutische annehmbare, nicht-toxische Säureadditionssalze mit geeigneten anorganischen Säuren, wie Chlorwasserstoffsäure, Bromwasserstoffsäure, Schwefelsäure oder Phosphorsäure, oder mit geeigneten organischen aliphatischen, cycloaliphatischen, aromatischen, araliphatischen oder heterocyclischen Carbon- oder Sulfonsäuren, wie Ameisensäure, Essigsäure, Propionsäure, Bernsteinsäure, Glykolsäure, Milchsäure, Äpfelsäure, Weinsäure, Zitronensäure, Ascorbinsäure, Maleinsäure, Fumarsäure, Brenztraubensäure, Benzoesäure, Anthranilsäure, 4-Hydroxybenzoesäure, Salicylsäure, Phenylessigsäure, Embonsäure, Methansulfonsäure, Aethansulfonsäure, Hydroxyäthansulfonsäure, Aethylensulfonsäure, 4-Chlorbenzolsulfonsäure, Toluolsulfonsäure, Naphthalinsulfonsäure, Sulfanilsäure oder Cyclohexylaminsulfonsäure.

Infolge der engen Beziehungen zwischen den neuen Verbindungen in freier Form und in Form ihrer Salze sind unter den freien Verbindungen und unter den Salzen sinn- und zweckgemäss gegebenenfalls auch die entsprechenden Salze bzw. freien Verbindungen zu verstehen.

Die neuen Verbindungen besitzen wertvolle pharmakologische Eigenschaften. Die Hauptwirkung der neuen Verbindungen besteht in einer Stimulation cardialer β-Rezeptoren, die sich z.B. am Herzen als positiv inotrope oder positiv chronotrope Wirkung nachweisen lässt. So wird in Konzentrationen ab etwa 0,3 ng/ml am isolierten Meerschweinchenvorhof eine Steigerung der Frequenz und Konzentrationskraft bewirkt.

Die positiv isotrope und chronotrope Wirkung lässt sich auch in vivo (narkotisierte Katze) als Steigerung der maximalen Druckanstiegsgeschwindigkeit im linken Ventrikel (dp/dt) und der Herzfrequenz ab etwa 0,3 µg/kg i.v. nachweisen. Einzelne Verbindungen zeigen dabei eine deutlich bevorzugte inotrope Wirkung. Die neuen Verbindungen bewirken ausserdem eine Blockade adrenergischer α-Rezeptoren, die z.B. ab etwa 100 ng/ml als Hemmung der Noradrenalinkontraktion am isolierten Samenleiter der Ratte gezeigt werden kann. Durch die neuen Verbindungen wird ausserdem eine Blutdrucksenkung bewirkt, die ab etwa 0,003 mg/kg nach i.v. Applikation an der narkotisierten Katze gezeigt werden kann. Einige der neuen Verbindungen zeigen eine deutlich bevorzugte Stimulation der $\beta_1$-Rezeptoren des Herzens gegenüber den $\beta_2$-Rezeptoren in Blutgefässen und Trachea und sind demzufolge als cardioselektive β-Stimulatoren zu betrachten.

Die neuen Verbindungen können somit als β-Stimulatoren, insbesondere als positiv inotrop wirksame Mittel zur Behandlung der Herzinsuffizienz, allein oder in Kombination mit anderen Präparaten, wie z.B. Herzglycosiden, verwendet werden.

Überraschenderweise wurde nun gefunden, dass die neuen Verbindungen den strukturell sehr nahestehenden, aus der zitierten DE-OS 21 35 678 bekannten Substanzen bezüglich der stimulierenden Wirksamkeit auf β-adrenerge Rezeptoren überlegen sind, wie aus den folgenden Vergleichsversuchen hervorgeht:

Versuchsbericht

Folgende Verbindungen wurden zur Ermittlung ihrer stimulierenden Wirksamkeit auf beta-adrenerge Rezeptoren geprüft:

I.: α-[N-[2-(3-Carbamoyl-4-hydroxy-phenoxy)-1-methyl-äthyl]-aminomethyl]-4-hydroxy-benzylalkohol;

II: α-[N-[2-(4-Carbamoyl-phenoxy)-äthyl]-aminomethyl]-4-hydroxy-benzylalkohol; (bekannt aus Beispiel 5 auf Seite 17 der DE-OS 21 35 678);

III: DL-Isoproterenolsulfat

A) Positiv inotrope und chronotrope Effekte am isolierten Meerschweinchenvorhof.

Meerschweinchen beiderlei Geschlechts im Gewicht von 250–450 g wurden mit Urethan intraperitoreal (1.5 g/kg) narkotisiert unb die Vorhöfe in einem Organbad montiert. An den rechten, spontan schlagenden und an den linken, mit konstanter Frequenz von 2.5 Hz elektrisch gereizten Vorhöfen wurden die Frequenz der rechten und die Kontraktionskraft der linken Vorhöfe kontinuierlich registriert, (isometrische Kontraktion unter 0.5 g diastolischer Vorspannung). Das Organbad enthielt 50 ml Krebs-Henseleit-Lösung von 32°C, oxygeniert mit einem Gemisch von 95% $O_2$ und 5% $CO_2$. Die Zugabe der Substanzen erfolgte kumulativ.

B) Positiv inotrope und chronotrope Effekte und Beeinflussung des Blutdruckes an der narkotisierten Katze

Katzen beiderlei Geschlechts wurden mit Nembutal® narkotisiert (35 mg/kg i.p. + 5 mg/kg/Std. i.v.) und bilateral vagotomiert. Ein Mikrotop-Durchtransducer (Miller C 350/5F) wurde durch die rechte A carotis in den linken Ventrikel vorgeschoben. Folgende Messparameter wurden kontinuierlich registriert: linksventrikulärer Druck, dP/dt im linken Ventrikel, mittlerer arterieller Blutdruck (rechte A. axillaris) und Herzfrequenz. Die Verbindungen wurden in steigenden Dosen bis zum Maximaleffekt intravenös verabreicht. Am Schluss der Versuche wurden steigende Dosen von DL-Isoproterenol-Sulfat i.v. bis zum Erreichen des Maximaleffekts gegeben. Die Maximalwirkungen der Substanzen wurden in % des Isoproterenol-Maximums ausgedrückt und die Dosen für halbmaximale Wirkungen ($ED_{50}$) bestimmt.

Tabelle 1
Wirkungen auf die Kontraktionskraft und Frequenz isolierter Meerschweinchenvorhöfe

| Verbindung | Steigerung der Kontraktionskraft (= positiv inotrope Wirkung) | | Steigerung der Frequenz (= positiv chronotrope Wirkung) | | Verhältnis der Wirksamkeit Chronotropie: Inotropie |
|---|---|---|---|---|---|
| | Wirksamkeit ($EC_{50}$; ng/ml)1) | relatives Ausmass der Wirkung in % der Isopoterenol-Wirkung2) | Wirksamkeit ($EC_{50}$; ng/ml)1) | relatives Ausmass der Wirkung in % der Isoproterenol-Wirkung2) | |
| I. | 0,3 | 100 | 0,1 | 100 | 3:1 |
| II. | 60 | 85 | 30 | 75 | 2:1 |
| III. | 1,0 | 100 | 0,7 | 100 | 1:1 |

1) $EC_{50}$ = Konzentration, die halbmaximale Effekte hervorruft,
2) Maximaleffekt von Isoproterenol = 100%.

Tabelle 2
Wirkungen auf die maximale Druckanstiegsgeschwindigkeit im linken Ventrikel $dP/dt_{max}$, Herzfrequenz und Blutdruck an der narkotisierten Katze

| Verbindung | Steigerung von $dP/dt_{max}$ | | Herzfrequenzsteigerung | | Blutdruckabnahme | |
|---|---|---|---|---|---|---|
| | Wirksamkeit ($ED_{50}$; µg/kg i.v.[1]) | relatives Ausmass der Wirkung (% von Isoproterenol) | Wirksamkeit $ED_{50}$; µg/kg i.v. | relatives Ausmass der Wirkung (% von Isoproterenol) | Wirksamkeit $ED_{50}$; µg/kg i.v. | relatives Ausmass der Wirkung (% von Isoproterenol |
| I. | 0,03 | 100 | 0,05 | 100 | 0,3 | 60 |
| II. | 1 | 100 | 7,5 | 100 | ~120 | 60 |
| DL-Isoproterenol | 0,07 | 100 | 0,09 | 100 | 0,04 | 100 |

1) $ED_{50}$ = Dosis, die halbmaximale Effekte hervorruft;
2) Maximaleffekte von Isoproterenol = 100%

Diskussion:

A) Isolierte Meerschweinchenvorhöfe

Die inotrope Wirksamkeit der in Tabelle 1 aufgeführten, gemäss vorliegender Anmeldung herstellbaren Substanz I ist entsprechend dem reziproken Verhältnis der jeweiligen $EC_{50}$ etwa 200-fach stärker als diejenige der Vergleichssubstanz II. Das Ausmass der inotropen Stimulation der Substanz I ist stärker als das der Vergleichssubstanz II und entspricht im wesentlichen demjenigen einer vollen Stimulation durch Isoproterenol.

Die chronotrope Wirksamkeit der Verbindung I ist etwa 300-fach stärker als die der Vergleichssubstanz II, während das Ausmass der chronotropen Stimulation etwas höher als das der Vergleichssubstanz II ist.

Die Vergleichsverbindung II zeigt eine leicht bevorzugte Stimulation chronotroper Rezeptoren, wie aus dem Verhältnis der Wirksamkeit Chronotropie:

Inotropie = 2:1 ersichtlich ist. Dasselbe gilt für die Verbindung I.

B) Narkotisierte Katze

Die inotrope Wirksamkeit (Steigerung des dP/dt max) der in Tabelle 2 aufgeführten Substanz I ist etwa 30-fach stärker, die chronotrope Wirksamkeit etwa 150-fach stärker als die der Vergleichsverbindung II. Das Ausmass der Wirkungen am Herzen ist bei den Verbindungen I und II gleich und entspricht demjenigen einer vollen Stimulation durch Isoproterenol.

Die für eine Blutdruckabnahme benötigten Dosen liegen bei der Verbindung I mit Ausnahme von Isoproterenol deutlich höher als die am Herzen wirksamen Dosen, woraus hervorgeht, dass diese Verbindung im Gegensatz zu Isoproterenol cardioselektiv wirkt. Als weiterer Ausdruck der cardioselektiven Wirksamkeit der geprüften Verbindungen ist die Tatsache zu werten, dass das Ausmass der mit dieser erreichten Blutdruckabnahme geringer ist als das durch Isoproterenol verursachte.

Die Erfindung betrifft insbesondere Verbindungen der Formel I, worin Ar jeweils unsubstituiertes oder durch 1 oder 2-Hydroxygruppen substituiertes Phenyl oder Pyridyl, z.B. 2- oder 3- oder 4-Pyridyl, oder Pyridazinyl z.B. 3- oder 4- oder 6-Pyridazinyl, oder Pyrimidinyl z.B. 2- oder 4- oder 5-Pyrimidinyl, oder Pyrazinyl, z.B. 2-Pyrazinyl, oder Furyl, z.B. 2- oder 3-Furyl, oder Pyrryl, z.B. 2- oder 3-Pyrryl, oder Thienyl, z.B. 2- oder 3-Thienyl, oder Oxazolyl, z.B. 2- oder 4-Oxazolyl, oder Thiazolyl, z.B. 2- oder 4-Thiazolyl, oder Pyrazolyl, z.B. 3- oder 5-Pyrazolyl, oder Imidazolyl, z.B. 2- oder 4-Imidazolyl, oder Thiadiazolyl wie 1,2,4-Thiadiazol-3- oder -5-yl oder 1,3,4-Thiadiazol-2-yl, und Alk einen Alkylenrest mit 2 bis 4 Kohlenstoffatomen bedeutet, wobei das Stickstoffatom und das Sauerstoffatom, oder, falls n für Null steht, der Phenylrest durch 2 bis 3 Kohlenstoffatome voneinander getrennt sind, und n für Null oder 1 steht, oder deren Salze, insbesondere deren pharmazeutisch annehmbaren nicht-toxischen Säureadditionssalze.

Die Erfindung betrifft in erster Linie Verbindungen der Formel I, worin Ar jeweils unsubstituiertes oder durch 1 oder 2 Hydroxygruppen substituiertes Phenyl, 2- oder 3-Pyridyl, 2- oder 3-Furyl oder 2- oder 3-Thienyl, und Alk einen Alkylenrest mit 2 bis 3 Kohlenstoffatomen bedeutet, wobei das Stickstoffatom und das Sauerstoffatom, oder, falls n für Null steht, der Phenylrest durch 2 bis 3 Kohlenstoffatome von einander getrennt sind, n für Null oder 1 steht, oder deren Salze, insbesondere pharmazeutisch annehmbaren nicht-toxischen Säureadditionssalze.

Die Erfindung betrifft ausserdem insbesondere folgende Verbindungen der Formel I, worin Ar jeweils unsubstituiertes oder durch 1 oder 2 Hydroxygruppen substituiertes Phenyl, 2- oder 3-Pyridyl, 2-Furyl oder 2-Thienyl und Alk einen Alkylenrest mit 2 bis 3 Kohlenstoffatomen bedeutet, und n für Null oder 1 steht, oder deren Salze, insbesondere deren pharmazeutisch annehmbaren nicht-toxischen Säureadditionssalze.

Die Erfindung betrifft speziell die in den Beispielen genannten Verbindungen der Formel I oder deren Salze, insbesondere deren pharmazeutisch annehmbaren, nicht-toxischen Säureadditionssalze.

Die neuen Verbindungen der Formel I werden in an sich bekannter Weise hergestellt. Man kann sie z.B. erhalten, indem man eine Verbindung der Formel

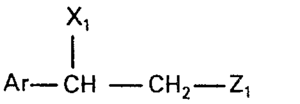

(II)

mit einer Verbindung der Formel

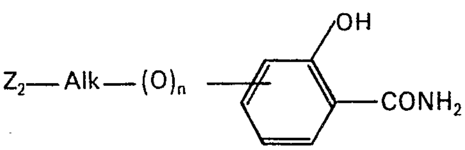

(III)

worin eine der Gruppen $Z_1$ und $Z_2$ eine reaktionsfähige veresterte Hydroxygrupe darstellt und die andere für die primäre Aminogruppe steht, und $X_1$ Hydroxy bedeutet, oder worin $X_1$ und $Z_1$ zusammen die Epoxygruppe bedeuten und $Z_2$ für die primäre Aminogruppe steht, Ar, Alk und n obige Bedeutung haben, umsetzt, und/oder, wenn erwünscht, eine erhaltene freie Verbindung in ein Salz oder ein erhaltenes Salz in eine freie Verbindung überführt, und/oder, wenn erwünscht, ein erhaltenes Isomerengemisch in die Isomeren oder ein erhaltenes Racemat in die Antipoden auftrennt.

Eine reaktionsfähige veresterte Hydroxygruppe $Z_1$ bzw. $Z_2$ ist eine, durch eine starke Säure, insbesondere eine starke anorganische Säure, wie eine Halogenwasserstoffsäure, insbesondere Chlor-, Brom- oder Jodwasserstoffsäure, oder Schwefelsäure, oder eine starke organische Säure, insbesondere eine starke organische Sulfonsäure, wie eine aliphatische oder aromatische Sulfonsäure, z.B. Methansulfonsäure, 4-Methylphenylsulfonsäure oder 4-Bromphenylsulfonsäure, veresterte Hydroxygruppe, und stellt in erster Linie Halogen, z.B. Chlor, Brom oder Jod, oder aliphatisch oder aromatisch substituiertes Sulfonyloxy, z.B. Methylsulfonyloxy oder 4-Methylphenylsulfonyloxy dar.

Die obige Reaktion wird in an sich bekannter Weise durchgeführt, wobei man, besonders bei Verwendung eines Ausgangsmaterials mit einer reaktionsfähigen veresterten Hydroxygruppe, vorteilhafterweise in Gegenwart eines basischen Mittels, wie einer anorganischen Base, z.B. eines Alkalimetall- oder Erdalkalimetallcarbonats oder -hydroxids, oder eines organischen basischen Mittels, wie eines Alkalimetall-niederalkanolats und/oder eines Überschusses des basischen Reaktionsteilnehmers und üblicherweise in Gegenwart eines Lösungsmittels oder Lösungsmittelgemisches und, wenn notwendig, unter Kühlen oder Erwärmen, z.B. in einem Temperaturbereich von etwa −20°C bis etwa +150°C, in einem offenen oder geschlossenen Gefäss und/oder in einer Inertgasatmosphäre, z.B. in einer Stickstoffatmosphäre, arbeitet.

Ausgangsstoffe der Formel II oder III sind bekannt oder können in an sich bekannter Weise hergestellt werden. So kann man eine Verbindung der Formel Ar-H, worin gegebenenfalls vorhandene Hydroxygruppen durch eine Schutzgruppe, z.B. eine der nachfolgend beschriebenen, geschützt sein können, mit einem Halogenacetylhalogenid, z.B. Chloracetylchlorid, in Gegenwart einer geeigneten Lewissäure, z.B. Aluminiumchlorid, nach der Friedel-Crafts-Methode an einem Kohlenstoffatom des Restes Ar halogenacetylieren und in der so erhältlichen Ar-Halogenacetyl-Verbindung, z.B. durch Behandeln mit einem geeigneten Hydridreduktionsmittel, die Carbonyl- zur Carbinolgruppe reduzieren; wenn erwünscht, kann man ein Halogen $Z_1$ z.B. durch Behandeln mit Ammoniak oder einem geeigneten Derivat davon, wie Hexamethylentetramin und Zersetzung der erhaltenen Verbindung mit verdünnter Mineralsäure, oder durch Umsetzen mit einem Alkalimetallsalz des Phthalimids und Spalten der erhaltenen N-Phthalimid-Verbindung, z.B. mit Hydrazin, in die primäre Aminogruppe $Z_1$ umwandeln. Ausgangsstoffe der Formel II, worin $X_1$ und $Z_1$ zusammen Epoxy bedeuten, können z.B. durch Cyclisierung einer Verbindung der Formel II, worin $H_1$ Hydroxy und $Z_1$ eine reaktionsfähige veresterte Hydroxygruppe, etwa Chlor oder Methansulfonyloxy darstellen, mittels alkalischer Reagentien, z.B. eines Gemischs von verdünnter Natronlauge und Tetrabutylamoniumchlorid in einem geeigneten Lösungsmittel, z.B. Methylenchlorid, erhalten werden. Ausgangsstoffe der Formel III können z.B. durch Umsetzung eines Hydroxysalicylamids mit einem der Bedeutung von Alk entsprechenden Dihalogenalkan, etwa einem Chlor-brom- oder Dibromalkan in Gegenwart eines alkalischen Kondensationsmittels, wie einem Alkalicarbonat, erhalten werden. Diese Umsetzungen werden in üblicher Weise vorgenommen, wobei an den Hydroxygruppen stehende Schutzgruppen gleichzeitig oder, wie nachfolgend beschrieben, abgespalten werden.

Die Verbindungen der Formel I können weiterhin hergestellt werden, indem man in einer Verbindung der Formel

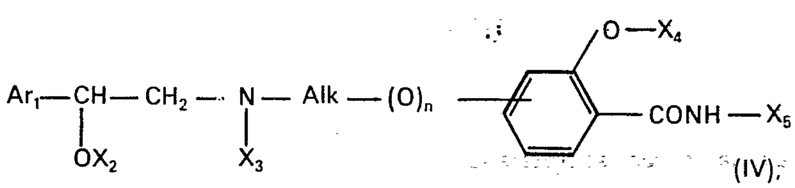

(IV);

worin $Ar_1$ die Bedeutung von Ar hat oder einen Rest Ar bedeutet, der durch 1 bis 2 in Hydroxy überführbare Gruppen substituiert ist, $X_2$, $X_3$ und $X_4$ jeweils Wasserstoff oder einen durch Wasserstoff ersetzbaren Substituenten bedeuten und $X_5$ für Wasserstoff steht, oder $X_2$ und $X_3$ und/oder $X_4$ und $X_5$ zusammen einen zweiwertigen, durch zwei Wasserstoffatome ersetzbaren Rest bedeuten mit der Massgabe, dass mindestens einer der Reste $X_2$, $X_3$ und $X_4$ von Wasserstoff verschieden ist, oder mindestens $Ar_1$ einen Rest Ar bedeutet, der durch 1 bis 2 in Hydroxy überführbare Gruppen substituiert ist, oder mindestens $X_2$ und $X_3$ zusammen oder $X_4$ und $X_5$ zusammen einen zweiwertigen, durch zwei Wasserstoffatome ersetzbaren Rest darstellen, oder in einem Salz davon, das von Wasserstoff verschiedene $X_2$, $X_3$, $X_4$ oder $X_4$ und $X_5$ zusammen durch Wasserstoff ersetzt, und/oder in einem Rest $Ar_1$ vorhandenes substituiertes Hydroxy in freies Hydroxy überführt, und wenn erwünscht, die zusätzlichen Verfahrensschritte durchführt.

Die Abspaltung der Gruppen $X_2$, $X_3$ oder $X_4$ oder jeweils $X_2$ und $X_3$ oder $X_4$ und $X_5$ zusammen sowie der in einem Rest $Ar_1$ vorhandenen Hydroxy-Substituenten wird mittels Solvolyse, wie Hydrolyse, Alkoholyse oder Acidolyse, oder mittels Reduktion einschliesslich Hydrogenolyse vorgenommen.

Eine besonders geeignete, abspaltbare Gruppe $X_3$ und $X_4$ sowie Hydroxy-Schutzgruppen in einem Rest $Ar_1$ ist in erster Linie eine hydrogenolytisch abspaltbare α-Arylniederalkylgruppe, wie eine gegebenenfalls substituierte 1-Polyphenyl- oder 1-Phenylniederalkylgruppe, worin Substituenten, insbesondere des Phenylteils, z.B. Niederalkyl, wie Methyl, oder Niederalkoxy wie Methoxy sein können, und in erster Linie Benzyl. Eine Gruppe $X_3$ und insbesondere $X_2$ und $X_4$ sowie Hydroxy-Schutzgruppen in einem Rest $Ar_1$ können auch einen solvolytisch, wie hydrolytisch oder acidolytisch, ferner einen reduktiv, einschliesslich hydrogenolytisch, abspaltbaren Rest, insbesondere einen entsprechenden Acylrest, wie den Acylrest einer organischen Carbonsäure, z.B. Niederalkanoyl, wie Acetyl, oder Aroyl, wie Benzoyl, ferner den Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenyl-niederalkylgruppe, worin Substituenten, in erster Linie de Phenylteils, z.B. die oben gegebene Bedeutung haben, und in erster Linie Trityl darstellen.

Ein durch $X_2$ und $X_3$ und/oder $X_4$ und $X_5$ zusammen gebildeter, abspaltbarer Rest ist in erster Linie eine hydrogenolytisch abspaltbare Gruppe, wie gegebenenfalls substituiertes 1-Phenylniederalkyliden, worin Substituenten, insbesondere des Phenylteils, z.B. Niederalkyl oder Niederalkoxy sein können, und insbesondere Benzyliden, sowie solvolytisch, insbesondere hydrolytisch abspaltbare Gruppen, wie Niederalkyliden, z.B. Methylen oder Isopropyliden, oder 1-Phenyl-niederalkyliden, dessen Phenylteil gegebenenfalls durch Niederalkyl, wie Methyl oder Niederalkoxy, wie Methoxy, substituiert ist, insbesondere Benzyliden oder Cycloalkyliden, z.B. Cyclopentyliden oder Cyclohexyliden.

In der Form von Salzen verwendbare Ausgangsstoffe werden in erster Linie in der Form von Säureadditionssalzen, z.B. mit Mineralsäuren, sowie mit organischen Säuren verwendet.

Hydrogenolytisch abspaltbare Reste $X_2$, $X_3$ und/oder $X_4$, insbesondere gegebenenfalls substituierte 1-Phenylniederalkylgruppen, ferner auch geeignete Acylgruppen, wie gegebenenfalls substituiertes 1-Phenylniederalkoxycarbonyl, sowie durch die Gruppen $X_2$ und $X_3$ sowie $X_4$ und $X_5$ zusammen gebildete, gegebenenfalls substituierte 1-Phenylniederalkylidengruppen, sowie in einem Rest $Ar_1$ vorhandene Hydroxy-Schutzgruppen dieser Art können durch Behandeln mit katalytisch aktiviertem Wasserstoff, z.B. mit Wasserstoff in Gegenwart eines Nickelkatalysators, wie Raney-Nickel, oder eines geeigneten Edelmetallkatalysators abgespalten werden.

Hydrolytisch abspaltbare Gruppen $X_2$, $X_3$ und/oder $X_4$ wie Acylreste von organischen Carbonsäuren, z.B. Niederalkanoyl, und Halbestern der Kohlensäure, z.B. Niederalkoxycarbonyl, ferner z.B. Tritylreste, sowie durch die Reste $X_2$ und $X_3$ und/oder $X_4$ und $X_5$ zusammen gebildete Niederalkyliden-, 1-Phenyl-niederalkyliden- oder Cycloalkylidengruppen sowie in einem Rest $Ar_1$ vorhandene Hydroxy-Schutzgruppen dieser Art können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxids oder -carbonats oder eines Amins wie Isopropylamin, abgespalten werden.

Acidolytisch abspaltbare Reste $X_2$, $X_3$ und/oder $X_4$ und/oder Hydroxy-Schutzgruppen in einem Rest $Ar_1$ sind insbesondere gewisse Acylreste von Halbestern der Kohlensäure, wie z.B. tert.-Niederalkoxycarbonyl oder gegebenenfalls substituierte Diphenylmethoxycarbonylreste, ferner auch tert.-Niederalkylreste; diese können durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäure (wenn notwendig, in Gegenwart eines aktivierenden Mittels, wie Anisol), sowie mit Ameisensäure abgespalten werden.

Unter reduktiv abspaltbaren Resten $X_2$, $X_3$ und/oder $X_4$ und/oder Hydroxy-Schutzgruppen in einem Rest $Ar_1$ werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl oder Arylmethoxycarbonyl, die z.B. beim Behandeln mit einem reduzierenden Schwermetall, wie Zink, oder mit einem reduzierenden Schwermetallsalz, wie einem

Chrom(II)salz, z.B. -chlorid oder -acetat, üblicherweise in Gegenwart einer organischen Carbonsäure, wie Ameisensäure oder Essigsäure, und von Wasser abgespalten werden können.

Schutzgruppen, welche an in einem Rest $Ar_1$ gegebenenfalls vorhandenen Hydroxygruppen stehen, entsprechen den vorhin genannten und mittels der beschriebenen Methoden abspaltbaren und durch Wasserstoff ersetzbaren Gruppen, wobei solche Gruppen im Zuge des beschriebenen Verfahrens gleichzeitig mit anderen Gruppen oder nachfolgend in einer getrennten Verfahrensmassnahme abgespalten werden.

Die obigen Reaktionen werden üblicherweise in Gegenwart eines Lösungsmittels, oder Lösungsmittelgemisches durchgeführt, wobei geeignete Reaktionsteilnehmer gleichzeitig auch als solche funktionieren können, und, wenn notwendig, unter Kühlen oder Erwärmen, z.B. in einem offenen oder geschlossenen Gefäss und/ oder in der Atmosphäre eines Inertgases, z.B. Stickstoff.

Die Ausgangsstoffe der Formel IV lassen sich in an sich bekannter Weise erhalten, indem man eine Verbindung der Formel $Ar_1$-H mit einem Halogenacetylhalogenid, z.B. Chloracetylchlorid, in Gegenwart einer geeigneten Lewissäure, z.B. Aluminiumchlorid, nach dem Friedel-Crafts-Verfahren umsetzt, die Carbonylgruppe in der so erhaltenen oder in anderer, üblicher Weise erhaltenen $Ar_1$-Halogenacetyl-Verbindung z.B. mittels Natriumborhydrid zur Carbinolgruppe reduziert und die erhaltene Verbindung mit einem Amin der Formel

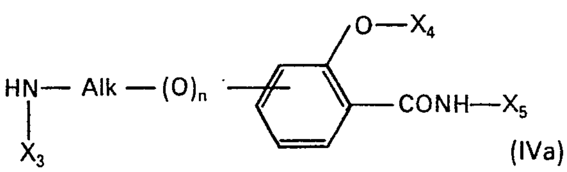

(IVa)

worin $X_3$ die angegebene Bedeutung hat, und $X_4$ oder $X_4$ und $X_5$ zusammen verschieden von Wasserstoff sind, umsetzt.

Man kann ferner z.B. die durch Umsetzung einer Verbindung der Formel

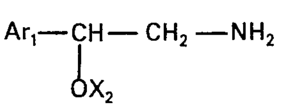

(IVb)

mit einer Carbonylverbindung der Formel

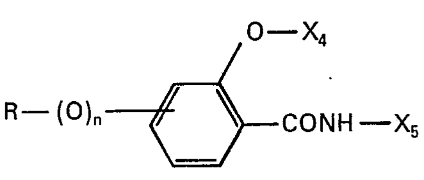

(IVc)

worin R einen dem Alkylenrest Alk entsprechenden, eine vom Sauerstoffatom bzw. Phenylrest durch mindestens ein Kohlenstoffatom getrennte Carbonylgruppierung enthaltenden Alkylrest bedeutet, und $X_4$ oder $X_4$ und $X_5$ zusammen eine der angegebenen Schutzgruppen bedeuten, gebildete Schiff'sche Base mit einem Borhydrid, etwa Natriumborhydrid zur Verbindung der Formel IV reduzieren. Die Reduktion kann auch mittels aktiviertem Wasserstoff in Gegenwart eines Hydrierkatalysators, z.B. eines Platin-auf-Kohle-Katalysators erfolgen.

Carbonylverbindungen der Formel (IVc) wiederum können durch Umsetzung einer Verbindung der Formel

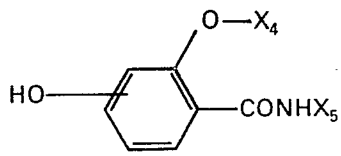

(IVd)

mit einer Verbindung der Formel R-Hal (IVe), worin R obige Bedeutung hat, und eine Verbindung (IVe) z.B. ein Halogenketon, z.B. Chloraceton darstellt, in üblicher Weise erhalten werden.

Die neuen Verbindungen der Formel I können ebenfalls erhalten werden, indem man in einer Verbindung der Formel

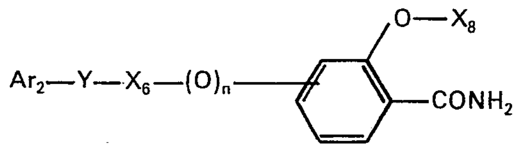

(V)

worin $X_6$ eine reduzierbare Gruppe der Formeln -CH = N - Alk- (Va), bzw. $-CH_2-N = Alk_1$ (Vb) oder $-C(=X_7)-N(X_8)-Alk-$(Vc) bzw. $-CH_2-N(X_8)-C(=X_7)-Alk_2-$(Vd) oder eine Gruppe $-CH_2-N(X_8)-Alk-$ (Ve) ist, wobei $Alk_1$ für den einem Rest Alk entsprechenden Alkyl-ylidenrest steht und $Alk_2$ einem um eine an das Stickstoffatom gebundene Methylengruppe verkürzten Rest Alk entspricht, $X_7$ den Oxo- oder Thioxorest und $X_8$ Wasserstoff oder einen unter den Bedingungen für die Reduktion von $X_6$ und/oder Y durch Wasserstoff ersetzbaren Rest darstellt und Y für einen Rest der Formel -CO- (Vf) oder $-CH(OX_8)-$ (Vg) steht, in der $X_8$ die vorstehend angegebene Bedeutung hat, $Ar_2$ einem Rest Ar entspricht, jedoch gegebenenfalls anstelle von einem oder zwei Hydroxy eine oder zwei Gruppen $OX_8$, in welchem $X_8$ die vorstehend angegebene Bedeutung hat, trägt, und n Null oder 1 bedeutet, wobei stets $X_6$ eine reduzierbare Gruppe Va bis Vd und/oder Y eine Carbonylgruppe Vf ist, diese Gruppe(n) reduziert und im gleichen Arbeitsgang die von Wasserstoff verschiedenen Gruppen $X_8$ durch Wasserstoff ersetzt, und, wenn erwünscht, die zusätzlichen Verfahrensschritte durchführt.

Eine hydrogenolytisch abspaltbare Gruppe $X_8$ ist in erster Linie eine α-Arylniederalkylgrupe, wie eine gegebenenfalls substituierte 1-Phenylniederalkylgruppe, worin Substituenten, z.B. Niederalkoxy, wie Methoxy sein können, und ganz besonders Benzyl.

Schutzgruppen, die an den den Rest $Ar_2$ gegebenenfalls substituierten Hydroxygruppen stehen, entsprechen den vorhin für $X_8$ genannten und mittels der beschriebenen Methoden abspaltbaren und durch Wasserstoff ersetzbaren Gruppen, wobei solche Gruppen im Zuge des beschriebenen Verfahrens gleichzeitig mit anderen Gruppen oder nachfolgend in einer getrennten Verfahrensmassnahme abgespalten werden.

Ausgangsstoffe der Formel V mit einer Gruppe $X_6$ der Formel Vb können auch in der isomeren Form von Ring-Tautomeren der Formel

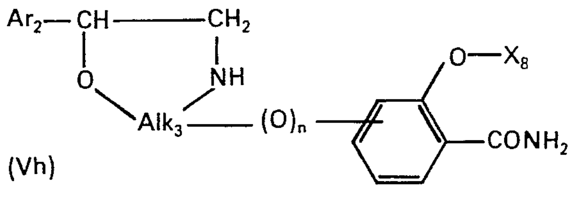

(Vh)

worin $Alk_3$ der Bedeutung von $Alk_1$ entspricht und das Sauerstoff- und Stickstoffatom an das gleiche Kohlenstoffatom gebunden sind, vorliegen.

Eine Alkyl-ylidengruppe $Alk_1$ ist z.B. Methin oder Acethyl-yliden, während eine Alkylidengruppe $Alk_2$ z.B. Methylen, Aethyliden oder 1-Methyl-äthyliden darstellt.

Die Reduktion der Stickstoff-Kohlenstoff-Doppelbindung in Ausgangsstoffen der Formel V, die als $X_6$ eine Gruppe Va oder Vb enthalten, während $Ar_2$, Y, $X_8$ und n die unter der Formel V angegebene Bedeutung haben (oder in den isomeren Verbindungen der Formel Vg der Sauerstoff-Kohlenstoff-Stickstoff-Bindung) zur Stickstoff-Kohlenstoff-Einfachbindung kann in an sich bekannter Weise z.B. durch Behandeln mit katalytisch aktiviertem Wasserstoff, wie Wasserstoff in Gegenwart eines geeigneten Hydrierkatalysators, z.B. eines Nickel-, Platin- oder Palladiumkatalysators erfolgen, wobei hydrogenolytisch abspaltbare Gruppen $X_8$ zugleich abgespalten und durch Wasserstoff ersetzt werden; oder man arbeitet mit einem geeigneten Hydridreduktionsmittel, wie einem Alkalimetallborhydrid, z.B. Natriumborhydrid. In allen Fällen wird zugleich mit der Gruppe Va oder Vb auch, falls vorhanden, ein Carbonylrest Y zum Hydroxymethylenrest reduziert, und bei Anwendung von Hydridreduktionsmitteln können auch an Sauerstoff gebundene Acylreste von Carbonsäuren, wie z.B. Essigsäure, als Reste $X_8$ vorliegen und im gleichen Arbeitsgang abgespalten werden.

Die Reduktion der Carbonylgruppe Y in Ausgangsstoffen der allgemeinen Formel V, die als Rest $X_6$ eine Gruppe Ve enthalten, während $Ar_2$, $X_8$ und n die unter der Formel V angegebene Bedeutung haben, kann in der vorstehend für Reduktion der Gruppen Va und Vb angegebenen Weise erfolgen, wobei wiederum bei der katalytischen Hydrierung entsprechende Reste $X_8$ hydrogenytisch abgespalten werden können.

Insbesonders geeignet zur Reduktion von Verbindungen der Formel V mit einer Gruppe der Formel Ve oder Vd und den unter der Formel V definierten Bedingungen von $Ar_2$, Y und n sind Hydridreduktionsmittel, wie z.B. Natriumborhydrid, oder Diboran.

Zugleich mit der Reduktion einer Gruppe Vc oder Vd erfolgt die Reduktion einer Carbonylgruppe Y, falls diese vorhanden ist, sowie die Abspaltung von an Sauerstoff gebundenen Acylresten von Carbonsäuren, wie z.B. Essigsäure, als Reste $X_8$. Andererseits ist durch Beschränkung der Menge Reduktionsmittel und geeignete Wahl der Reduktionsbedingungen dafür zu sorgen, dass die aromatisch gebundene Carboxamidgruppe nicht reduziert wird. Gruppierungen der Formeln Ve und Vd, worin $X_7$ jeweils eine Thioxogruppe bedeutet, werden durch reduktive Entschwefelung, z.B. durch Behandeln mit einem Hydrierkatalysator, wie Raney-Nickel, in die Gruppierung der Formel $-CH_2-NH-Alk-$ umgewandelt. Die obigen Reduktionsreaktionen werden in an sich bekannter Weise, üblicherweise in Gegenwart eines inerten Lösungsmittels, und, wenn notwendig, unter Kühlen oder Erwärmen, z.B. in einem Temperaturbereich von etwa $-20°C$ bis etwa $+150°C$, und/oder in einem geschlossenen Gefäss unter Druck und/oder in einer Inertgas-, z.B. Stickstoffatmosphäre, durchgeführt.

Ein Ausgangsmaterial der Formel V kann in an sich bekannter Weise, gegebenenfalls in situ, d.h. unter den Bedingungen des beschriebenen Verfahrens, hergestellt werden. so kann man eine Verbindung der Formel $Ar_2$-H (Vi) mit einem Essigsäurehalogenid oder -anhydrid in Gegenwart einer Lewissäure acetylieren und in dem erhaltenen Zwischenprodukt die Acetylgruppe dann z.B. durch Behandeln mit einem geeigneten Oxidationsmittel, wie Selendioxid, in die Glyoxyloylgruppe umwandeln. Eine solche Glyoxylverbindung, oder, wenn erwünscht, ein geeignetes Derivat derselben, etwa ein Acetal, kann dann mit einem Amin der Formel

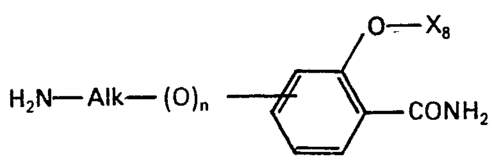

(Vj)

zu einem Ausgangsprodukt der Formel V mit der Gruppe $X_6$ der Formel Va umgesetzt werden, worin Y die Carbonylgruppe darstellt. Man kann ferner eine Verbindung der Formel (Vi) mit einem Halogenacetylhalogenid, z.B. Chloracetylchlorid, in Gegenwart einer geeigneten Lewissäure, z.B. Aluminiumchlorid, nach Friedel-Crafts-Methode zur entsprechenden $Ar_2$-Chloracetyl-Verbindung halogenacetylieren, in der so oder in anderer üblicher Weise erhältlichen Halogenacetylverbindung durch Behandeln mit einem geeigneten Hydridreduktionsmittel die Carbonyl- zur Carbinolgruppe reduzieren, und das Halogenatom durch Behandeln mit Ammoniak oder einem geeigneten Derivat davon, wie Hexamethylentetramin und Zersetzen des gebildeten Reaktionsproduktes mit verdünnter Säure, etwa wässriger Salzsäure, in die primäre Aminogruppe umwandeln.

Durch Umsetzung des so erhältlichen Zwischenprodukts der Formel

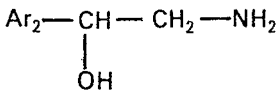 (Vk)

mit einer Carbonylverbindung der Formel

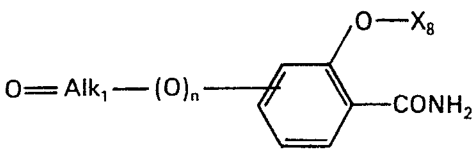 (VI)

kann man zu Ausgangsstoffen der Formel V mit einer Gruppe $X_6$ der Formel (Vb) gelangen. Eine Modifizierung dieser Umsetzungen besteht darin, dass man in dem vorhin beschriebenen Zwischenprodukt das Halogenatom, statt es durch Behandeln mit Ammoniak etc. gegen die primäre Aminogruppe auszutauschen, durch Umsetzung mit einem 1-Aryl-niederalkylamin, z.B. Benzylamin oder einem Di-(1-aryl-niederalkyl)-amin z.B. Dibenzylamin gegen die entsprechende 1-Arylniederalkylamino bzw. Di-(1-arylniederalkyl)-aminogruppe austauscht und die erhaltene Verbindung, etwa die entsprechende Dibenzylaminoverbindung, mit der Oxoverbindung der Formel (V1) unter den reduzierenden Bedingungen des Verfahrens umsetzt. Hierbei verwendet man als Reduktionsmittel in erster Linie katalytisch aktivierten Wasserstoff, z.B. Wasserstoff in Gegenwart eines Schwermetallhydrierkatalysators oder eines Gemisches davon, wie eines Palladium- und/oder Platinkatalysators. Unter solchen Reaktionsbedingungen werden hydrogenolytisch abspaltbare Gruppen $X_8$, z.B. Benzylgruppen abgespalten, die gegebenenfalls vorhandene Carbonyl- zur Carbinolgruppe und gleichzeitig die Stickstoff-Kohlenstoff-Doppelbindung zur entsprechenden Stickstoff-Kohlenstoff-Einfachbindung reduziert.

Oxoverbindungen der Formel (V1) wiederum, worin n für 1 steht, sind z.B. durch Umsetzung einer Dihydroxyverbindung der Formel

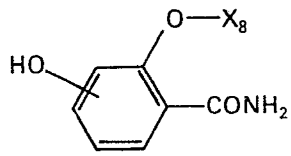 (Vm)

mit einer Halogenalkanon-Verbindung der oben erläuterten Formel R - Hal (IVe), z.B. Chloraceton, in Gegenwart eines alkalischen Kondensationsmittels, etwa Kaliumcarbonat, oder einer organischen Base wie Triäthylamin, erhältlich.

Ausgangsprodukte der Formel V mit einer Gruppe $X_6$ der Formeln Vc oder Vd können in an sich bekannter Weise hergestellt werden, indem man z.B. eine Formylverbindung der Formel $Ar_2$ - CHO (Vn) mit Cyanwasserstoff umsetzt und im so erhältlichen Cyanhydrin-Zwischenprodukt die Cyan- zur Carboxylgruppe, z.B. unter sauren Bedingungen, hydrolysiert. Die so oder über die Zwischenstufen Imidchlorid, Imido-niederalkylester und Niederalkylester erhaltene $Ar_2$-2-hydroxyessigsäure wird dann in Gegenwart eines geeigneten Kondensationsmittels, z.B. eines Carbodiimids, wie Dicyclohexylcarbodiimid, mit einem Amin der Formel (Vj) umsetzt, wonach man ein Ausgangsmaterial der Formel V mit der Gruppe $X_6$ der Formel (Vc) erhält.

Ferner kann man durch Umsetzung einer Verbindung der Formel Vk mit einer Verbindung der Formel

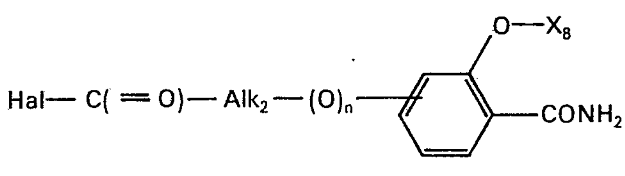 (Vo)

worin Hal für Halogen und insbesondere für Chlor steht, ein Ausgangsmaterial der Formel (V) mit der Gruppe $X_6$ der Formel (Vd) erhalten. Man kann ferner in der oben angegebenen $Ar_2$- Chloracetylverbindung das Chlor etwa durch Umsetzung mit Hexamethylentetramin gegen die primäre Aminogruppe austauschen und eine so erhaltene Verbindung mit einer Halogenverbindung der Formel (Vo) umsetzen. In einem so erhaltenen Ausgangsprodukt der Formel V mit einer Gruppe $X_6$ der Formel Vd und worin Y die Carbonylgruppe darstellt, kann man die Carbonyl- zur Carbinolgruppe und die aliphatisch gebundene Carbamoylgruppe zur Gruppe der Formel

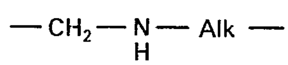

gleichzeitig reduzieren, etwa mittels eines Hydridreduktionsmittels, insbesondere Diboran.

Die neuen Verbindungen der Formel I können ebenfalls erhalten werden, indem man eine Verbindung der Formel

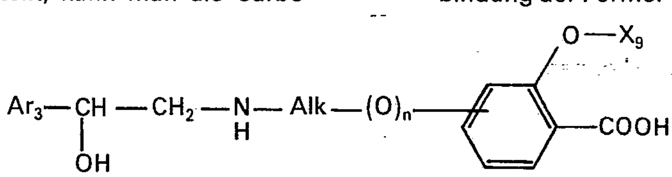 (VI)

worin $Ar_3$ die Bedeutung von Ar hat, oder einen Rest Ar darstellt, der durch 1 bis 2 mittels Ammonolyse in Hydroxy überführbare Gruppen substituiert ist, $X_9$ Wasserstoff oder eine mittels Ammonolyse abspaltbare Gruppe bedeutet, oder ein reaktionsfähiges Derivat einer der in Formel VI definierten Carbonsäuren, mit Ammoniak umsetzt, und gleichzeitig gegebenenfalls vorhandene Reste $X_9$ abspaltet und durch Wasserstoff ersetzt.

Hydrolytisch und insbesondere ammonolytisch abspaltbare Reste $X_9$ sind Acylreste von organischen Carbonsäuren, z.B. Aroyl wie Benzoyl oder Niederalkanoyl, wie Acetyl.

Reaktionsfähige Derivate der in Formel VI definierten Carbonsäuren sind z.B. die Halogenide, wie die Chloride oder Bromide, ferner die Azide, sowie Säureanhydride insbesondere gemischte Säureanhydride mit z.B. Niederalkancarbonsäuren, wie Essigsäure oder Propionsäure, Niederalkoxyalkancarbonsäuren, wie 2-Methoxyessigsäure. Reaktionsfähige Derivate der Formel VI sind insbesondere Ester, z.B. mit Niederalkanolen, wie Methanol, Äthanol, Isopropanol, tert.-Butanol, ferner mit Arylniederalkanolen, etwa gegebenenfalls durch Niederalkyl, z.B. Methyl oder Niederalkoxy z.B. Methoxy, substituierter Benzylalkohol, oder Phenolen, die gegebenenfalls durch geeignete Substituenten aktiviert sind, z.B. durch Halogen, etwa 4-Halogen, wie 4-Chlor, Niederalkoxy etwa 4-Niederalkoxy wie 4-Methoxy, 4-Nitro oder 2,4-Dinitro, wie etwa 4-Chlorphenol, 4-Methoxyphenol, 4-Nitro- oder 2,4-Dinitrophenol, ferner mit Cycloalkanolen, wie etwa Cyclopentanol oder Cyclohexanol, die gegebenenfalls durch Niederalkyl z.B. Methyl substituiert sein können. Die Umsetzung wird in an sich bekannter Weise, überlicherweise in Gegenwart eines inerten Lösungsmittels z.B. in einem Temperaturbereich von etwa −10° bis +50°C in einem geschlossenen Gefäss durchgeführt.

Die Ausgangsstoffe der Formel VI lassen sich in an sich bekannter Weise erhalten, indem man eine Verbindung der Formel $Ar_3 - COCH_3$ bromiert, die Carbonylgruppe in der so erhaltenen $Ar_3$-Halogenacetyl-Verbindung zur Carbinolgruppe, z.B. mittels Diboran, reduziert und diese mit einem Amin der Formel

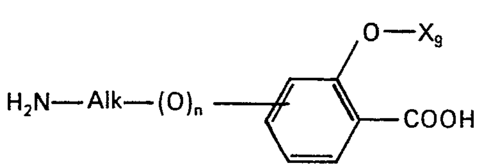 (VIa)

worin $X_9$ die angegebene Bedeutung hat, oder einem reaktionsfähigen Derivat davon, umsetzt. Man kann auch die $Ar_3$-Halogenacetyl-Verbindung mit dem Amin der Formel VIa umsetzen und nachträglich die Carbonyl- in die Carbinolgruppe umwandeln.

Man kann ferner die durch Umsetzung einer Verbindung der Formel

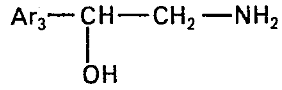 (VIb)

mit einer Carbonylverbindung der Formel

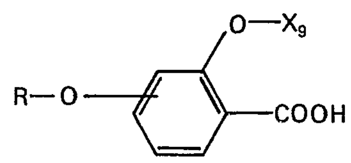 (VIc)

worin R dem einen Alkylenrest Alk entsprechenden, eine Carbonylgrupe enthaltenden, Alkylrest entspricht, gebildete Schiff'sche Base mit einem Borhydrid, etwa Natriumborhydrid reduzieren. Die Reduktion kann auch mittels aktiviertem Wasserstoff in Gegenwart eines Hydrierkatalysators, z.B. eines Platin-auf-Kohle-Katalysators erfolgen.

Carbonylverbindungen der Formel (VIc) wiederum, worin n 1 bedeutet, können durch Umsetzung einer Verbindung der Formel

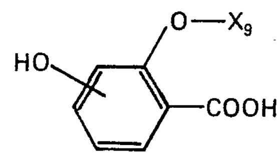

mit einer Verbindung der Formel R-Hal (IVe), worin Hal für Halogen, insbesondere Chlor steht, in an sich bekannter Weise erhalten werden.

Je nach den Verfahrensbedingungen und Ausgangsstoffen erhält man die neuen Verbindungen in freier Form oder in der ebenfalls von der Erfindung umfassten Form ihrer Salze, wobei die neuen Verbindungen oder Salze davon auch als Hemi-, Mono-, Sesqui- oder Polyhydrate davon vorliegen können. Säureadditionssalze der neuen Verbindungen können in an sich bekannter Weise, z.B. durch Behandeln mit basischen Mitteln, wie Alkalimetallhydroxiden, -carbonaten oder -hydrogencarbonaten oder Ionenaustauschern, in die freien Verbindungen übergeführt werden. Andererseits können erhaltene freie Basen mit organischen oder anorganischen Säuren, z.B. mit den genannten Säuren, Säureadditionssalze bilden, wobei zu deren Herstellung insbesondere solche Säuren verwendet werden, die sich zur Bildung von pharmazeutisch annehmbaren Salzen eignen.

Diese oder andere Salze, insbesondere Säureadditionssalze der neuen Verbindungen, wie z.B. Oxalate oder Perchlorate, können auch zur Reinigung der erhaltenen freien Basen dienen, indem man die freien Basen in Salze überführt, diese abtrennt und reinigt, und aus den Salzen wiederum die Basen freisetzt.

Die neuen Verbindungen können je nach der Wahl der Ausgangsstoffe und Arbeitsweisen, als optische Antipoden oder Racemate, oder sofern sie mindestens zwei asymmetrische Kohlenstoffatome enthalten, auch als Racematgemische vorliegen. Die Ausgangsstoffe können auch als optische Antipoden eingesetzt werden.

Erhaltene Racematgemische können auf Grund der physikalisch-chemischen Unterschiede der Diastereoisomeren in bekannter Weise, z.B. durch Chromatographie und/oder fraktionierte Kristallisation, in die beiden stereoisomeren (diastereomeren) Racemate aufgetrennt werden.

Erhaltene Racemate lassen sich nach an sich bekannten Methoden in die Antipoden zerlegen, z.B. durch Umkristallisieren aus einem optisch aktiven Lösungsmittel, durch Behandeln mit geeigneten Mikroorganismen oder durch Umsetzen mit einer, mit der racemischen Verbindung Salze bildenden optisch aktiven Substanz, insbesondere Säuren, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Säuren sind z.B. die D- und L-Formen von Weinsäure, Di-o-Toluolweinsäure, Äpfelsäure, Mandelsäure, Camphersulfonsäure, Glutaminsäure, Asparaginsäure oder Chinasäure. Vorteilhaft isoliert man den wirksameren der beiden Antipoden.

Die Erfindung betrifft auch diejenigen Ausführungsformen des Verfahrens, nach denen man von einer auf irgeneiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form ihrer Salze vorliegt.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Die Ausgangsstoffe sind bekannt oder können, falls sie neu sind, nach an sich bekannten Methoden, wie oben, z.B. analog wie in den Beispielen beschrieben, erhalten werden.

Die neuen Verbindungen können z.B. in Form pharmazeutischer Präparate Verwendung finden, welche eine pharmakologische wirksame Menge der Aktivsubstanz, gegebenenfalls zusammen mit pharmazeutisch verwendbaren Trägerstoffen enthalten, die sich zur enteralen, z.B. oralen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycerin und/oder Schmiermitteln, z.B. Kieselerde, Talk, Stearinsäure oder Salze davon, wie Magnesium- oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die neuen pharmakologisch wirksamen Verbindungen in Form von parenteral verabreichbaren Präparaten oder von Infusionslösungen verwenden. Solche Lösungen sind vorzugsweise isotonische wässrige Lösungen oder Suspensionen, wobei dieses z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes.

Die Dosierung kann von verschiedenen Faktoren, wie Applikationsweise, Spezies, Alter und/oder individuellen Zustand abhängen. So liegen die täglich zu verabreichenden Dosen bei oraler Applikation an Warmblütern von etwa 70 kg vorzugsweise zwischen etwa 0,005 und 0,1 g.

Die folgenden Beispiele dienen zur Illustration der Erfindung; Temperaturen werden in Celsiusgraden angegeben.

Beispiel 1

Eine Lösung von 40 g rohem α-[N-[2-(2,3-Dihydro-2,2-dimethyl-4-oxo-4H-1,3-benzoxazin-6-yl-oxy)-1-methyläthyl]-aminomethyl]-benzylalkohol in 450 ml Methanol wird mit 100 ml Isopropylamin versetzt und 1,5 Stunden unter Rückfluss gekocht. Nach dem Eindampfen des Reaktionsgemisches verbleibt ein Schaum, der in möglichst wenig Isopropanol heiss gelöst wird. Die gebildeten Kristalle werden nach mehrtägigem Stehen abgesaugt, welche ein Gemisch der Diastereoisomeren des α-[N-[2-(3-Carbamoyl-4-hydroxyphenoxy)-1-methyl-äthyl]-aminomethyl]-benzylalkohols mit einem Schmelzintervall von ca. 120–150° darstellen. Durch fraktioniertes Umkristallisieren aus Isopropanol erhält man die reinen Enantiomeren-Paare, welche bei 166–168° bzw. 152–154° schmelzen.

Der Ausgangsstoff wird auf folgende Weise hergestellt:

a) Nach der von Irvine et al., Synthesis 1972, 568 beschriebenen Methode wird 2,5-Dihydroxybenzamid unter Verwendung eines Überschusses von Aceton in 2,3-Dihydro-2,2-dimethyl-6-hydroxy-4H-1,3-benzoxazin-4-on vom Smp. 215–216° übergeführt.

b) 70 g 2,3-Dihydro-2,2-dimethyl-6-hydroxy-4H-1,3-benzoxazin-4-on werden in 400 ml Acetonitril mit 100 g Kaliumcarbonat und 32 ml Chloraceton 30 Stunden unter Rückfluss gerührt. Nach Zusatz von weiteren 3,2 ml Chloraceton wird das Reaktionsgemisch während weiteren 15–20 Stunden erhitzt. Das noch warme Reaktionsgemisch wird filtriert, der Rückstand mit Aceton gründlich gewaschen und das vereinigte Filtrat eingedampft. Der kristalline Rückstand wird aus Toluol umkristallisiert und ergibt das 2,3-Dihydro-2,2-dimethyl-6-(2-oxo-propoxy)-4H-1,3-benzoxazin-4-on vom Smp. 125–126°.

c) Eine Lösung von 26,6 g 2,3-Dihydro-2,2-dimethyl-6-(2-oxo-propoxy)-4H-1,3-benzoxazin-4-on und 14,6 g α-(Aminomethyl)-benzylalkohol in 450 ml Methanol wird unter Zusatz von 50 mg konz. Schwefelsäure und 4 g Platin-auf-Kohle-Katalysator bis zur Aufnahme von 1 Mol-Äquivalent Wasserstoff bei Normalbedingungen hydriert. Die nach dem Abfiltrieren des Katalysators erhaltene Lösung, die den rohen α-[N-[2-(2,3-Dihydro-2,2-dimethyl-4-oxo-4H-1,3-benzoxazin-6-yloxy)-1-methyl-äthyl]-aminomethyl]-benzylalkohol enthält, wird ohne Aufarbeitung hydrolysiert.

d) Die in Beispiel 1 verwendete Verbindung kann auch analog Beispiel 2c) durch Reduktion der aus dem gemäss Beispiel 1b erhaltenen Keton und α-(Aminomethyl)-benzylalkohol erhaltenen Schiffschen Base mittels Natriumborhydrid erhalten werden.

Beispiel 2

18 g roher α-[N-[2-(2,3-Dihydro-2,2-dimethyl-4-oxo-4H-1,3-benzoxazin-7-yloxy)-1-methyl-äthyl]-aminomethyl]-benzylalkohol wird in 100 ml Isopropanol gelöst, mit 50 ml Isopropylamin versetzt und 1,5 Stunden unter Rückfluss gekocht. Das Reaktionsgemisch wird nach dem Eindampfen zwischen 50 ml 2-n. Salzsäure und 100 ml Äther verteilt, die Wasserphase abgetrennt, mit konz. Ammoniaklösung alkalisch gestellt und 3 mal mit je 150 ml Äthylacetat extrahiert.

Nach dem Eindampfen erhält man einen Schaum, der nach Zugabe von Isopropanol kristallisiert. Man erhält α-[N-[2-(4-Carbamoyl-3-hydroxy-phenoxy)-1-methyläthyl]-aminomethyl]-benzylalkohol als Diastereomeren-Gemisch vom Schmelzintervall 140–156°. Durch fraktionierte Kristallisation aus Methanol erhält man die beiden Enantiomeren-Paare vom Smp. 171–173° bzw. 149–151°.

Der Ausgangsstoff wird auf folgende Weise hergestellt:

a) Aus 2,4-Dihydroxy-benzamid erhält man analog Beispiel 1a) das 2,3-Dihydro-2,2-dimethyl-7-hydroxy-4H-1,3-benzoxazin-4-on vom Smp. 249–251°.

b) Analog Beispiel 1b) erhält man aus 168 g 2,3-Dihydro-2,2-dimethyl-7-hydroxy-4H-1,3-benzoxazin-4-on, 305 g Kaliumcarbonat und 88 ml Chloraceton in 1,2 Liter Acetonitril durch Kochen während 28 Stunden und nachfolgenden Aufarbeiten das 2,3-Dihydro-2,2-dimethyl-7-(2-oxo-propoxy)-4H-1,3-benzoxazin-4-on vom Smp. 160–162° (aus Isopropanol).

c) Ein Gemisch von 13,0 g 2,3-Dihydro-2,2-dimethyl-7-(2-oxo-propoxy)-4H-1,3-benzoxazin-4-on und 6,9 g α-(Aminomethyl)-benzylalkohol wird mit 100 ml Äthanol 5 Stunden unter Rückfluss gekocht. Unter Eiskühlung und Rühren gibt man hierauf 5,7 g Natriumborhydrid portionsweise zu und rührt hierauf bei Raumtemperatur über Nacht. Unter Eiskühlung wird dann das überschüssige Natriumborhydrid mit 2-n. Salzsäure (ca. 75 ml) zersetzt und das Reaktionsgemisch eingeengt. Die wässrige Phase wird mit konz. Ammoniaklösung alkalisch gestellt und das ausgefällte Produkt zweimal mit je 500 ml Äthylacetat extrahiert.

Der durch Eindampfen erhaltene rohe α-[N-[2-(2,3-Dihydro-2,2-dimethyl-4-oxo-4H-1,3-benzoxazin-7-yl-oxy)-1-methyl-äthyl]-aminomethyl]-benzylalkohol wird ohne weitere Reinigung hydrolysiert.

Beispiel 3

Ein Gemisch von 25,4 g α-(Aminomethyl)-benzylalkohol und 11,1 g 2,3-Dihydro-2,2-dimethyl-6-(2-bromäthoxy)-4H-1,3-benzoxazin-4-on wird geschmolzen und 3 Stunden in einem Bad von 130° gerührt. Nach dem Abkühlen löst man das Reaktionsgemisch in 50 ml Isopropanol und verdünnt mit ca. 50 ml Äthylacetat, wobei der α-[N-[2-(3-Carbamoyl-4-hydroxy-phenoxy)-äthyl]-aminomethyl]-benzylalkohol vom Smp. 172–174° auskristallisiert. Nach Umkristallisation aus 300 ml Äthanol schmilzt das Produkt bei 174–175°.

Das Ausgangsmaterial wird auf folgende Weise hergestellt:

a) Ein Gemisch von 48,2 g 2,3-Dihydro-2,2-dimethyl-6-hydroxy-4H-1,3-benzoxazin-4-on, 70 g Kaliumcarbonat und 250 ml 1,2-Dibromäthan wird unter Rühren und Rückfluss während 4 Stunden gekocht. Das breiartige Reaktionsgemisch wird 3–4 mal mit je 1 Liter Methanol heiss extrahiert, die vereinigten Methanol-Extrakte eingedampft und der Rückstand aus Methanol umkristallisiert. Man erhält 55 g 2,3-Dihydro-2,2-dimethyl-6-(2-bromäthoxy)-4H-1,3-benzoxazin-4-on vom Smp. 190–195°.

Beispiel 4

Eine Lösung von 15 g rohem N-]2-(4-Carbamoyl-3-hydroxy-phenoxy)-äthyl]-N-(2-hydroxy-2-phenyl-äthyl)-benzylamin in 150 ml Methanol wird unter Zusatz von 3 g Palladium-auf-Kohle-Katalysator (5%) bis zum Stillstand unter Normalbedingungen hydriert. Der Katalysator wird abfiltriert, das Lösungsmittel abgedampft und der Rückstand aus Isopropanol umkristallisiert. Man erhält den α-[N-[2-(4-Carbamoyl-3-hydroxy-phenoxy)-äthyl]-aminomethyl]-benzylalkohol vom Smp. 141–142°. Die Verbindung bildet ein Hydrochlorid vom Smp. 240–242°.

Das Ausgangsmaterial wird auf folgende Weise hergestellt:

a) 16,2 g 2,3-Dihydro-2,2-dimethyl-7-hydroxy-4H-1,3-benzoxazin-4-on werden analog Beispiel 3a) mit 84 ml 1,2-Dibromäthan umgesetzt und ergeben das 2,3-Dihydro-2,2-dimethyl-7-(2-bromäthoxy)-4H-1,3-benzoxazin-4-on vom Smp. 156–158° (aus Isopropanol).

b) 53 g 2,3-Dihydro-2,2-dimethyl-7-(2-bromäthoxy)-4H-1,3-benzoxazin-4-on und 94 g Benzylamin werden unter Rühren 3 Stunden gekocht. Das Reaktionsgemisch wird mit konz. Ammoniak alkalisch gestellt, und die organische Phase bei max. 50° eingedampft.

Das so erhaltene N-[2-(4-Carbamoyl-3-hydroxy-phenoxy)-äthyl]-benzylamin bildet ein Öl, dessen Hydrochlorid bei 252–254° schmilzt (aus Methanol).

c) Ein Gemisch von 11,0 g Phenyläthylenoxid, 9,2 g N-[2-(4-Carbamoyl-3-hydroxy-phenoxy)-äthyl]-benzylamin und 100 ml Isopropanol wird 10 Stunden unter Rückfluss gekocht. Das Reaktionsgemisch wird eingedampft und der Rückstand mit 50 ml Hexan verrührt. Das im Hexan unlösliche Öl besteht im wesentlichen aus N-[2-(4-Carbamoyl-3-hydroxy-phenoxy)-äthyl]-N-(2-hydroxy-2-phenyl-äthyl)-benzylamin, das in rohem Zustand hydriert wird.

Beispiel 5

Eine Lösung von 13,0 g α-(Aminomethyl)-3-pyridinmethanol und 26,5 g 2,3-Dihydro-2,2-dimethyl-6-(2-oxo-propoxy)-4H-1,3-benzoxazin-4-on in 400 ml Methanol wird unter Zusatz von 0,1 ml konz. Schwefelsäure und 1 g Platin-auf-Kohle-Katalysator (5%) bei 30° und Normaldruck hydriert. Bis zur Aufnahme der berechneten Menge Wasserstoff sind 2 weitere Zusätze von jeweils 1 g bzw. 2 g des gleichen Katalysators erforderlich. Nach Abfiltrieren des Katalysators werden 50 ml Isopropylamin zum Filtrat gegeben, dieses 1 Stunde unter Rückfluss gekocht und eingedampft. Der Rückstand, ein dunkelbrauner Schaum, wird in 50 ml Methanol gelöst, mit 6,1 g Fumarsäure versetzt und bis zur Lösung erwärmt. Nach längerer Zeit kristallisiert das α-[N-[2-(3-Carbamoyl-4-hydroxy-phenoxy)-1-methyl-äthyl]-aminomethyl]-3-pyridinmethanol als neutrales Fumarat vom Smp. 192–196° (sintert ab 187°), aus, in welchem das eine Enantiomerenpaar stark angereichert ist (~ 80%).

Beispiel 6

Analog der im Beispiel 5 beschriebenen Verfahrensweise, jedoch unter Verwendung von 13,0 g α-(Aminomethyl)-2-pyridinmethanol erhält man das rohe α-[N-[2-(3-Carbamoyl-4-hydroxy-phenoxy)-1-methyl-äthyl]-aminomethyl]-2-pyridinmethanol als braunes Öl. Es bildet ein neutrales Fumarat (aus Methanol), welches bei 147–165° schmilzt und ein Gemisch der Diastereomeren darstellt.

Beispiel 7

Eine Lösung von 15,2 g 4-Benzyloxy-α-[N-[2-(3-carbamoyl-4-hydroxy-phenoxy)-1-methyl-äthyl]-aminomethyl]-benzylalkohol in 500 ml Methanol wird in Anwesenheit von 2 g Palladium-auf-Kohle-Katalysator (5%) bis zur Aufnahme von 1 Moläquivalent Wasserstoff bei Normalbedingungen hydriert. Der Katalysator wird abfiltriert und das Filtrat eingedampft. Der verbliebene Schaum wird zwischen 300 ml Äthylacetat und 50 ml Kaliumbicarbonat-Lösung verteilt, die organische Phase abgetrennt, getrocknet und eingedampft. Der so erhaltene, rohe α-[N-[2-(3-Carbamoyl-4-hydroxy-phenoxy)-1-methyl-äthyl]-aminomethyl]-4-hydroxybenzylalkohol bildet mit der halben äquivalenten Menge Fumarsäure in Methanol ein neutrales Fumarat, welches nach längerer Zeit aus Aceton als Diastereomerengemisch kristallisiert, Smp. 185–195°. Durch fraktionierte Kristallisation aus Methanol kann daraus ein einheitliches Enantiomerenpaar vom Smp. 209–212° (Zersetzung) erhalten werden.

Das Ausgangsmaterial wird auf folgende Weise hergestellt:

a) Eine Lösung von 9,6 g 2,3-Dihydro-2,2-dimethyl-6-(2-oxo-propoxy)-4H-1,3-benzoxazin-4-on und 9,37 g 4-Benzyloxy-α-(aminomethyl)-benzylalkohol in 250 ml Methanol wird analog Beispiel 1 bis zur Aufnahme von einem Äquivalent Wasserstoff hydriert. Der Katalysator wird abfiltriert und die Lösung nach Zugabe von 40 ml Isopropylamin 1 Stunde unter Rückfluss gekocht. Durch Eindampfen des Reaktionsgemisches erhält man den 4-Benzyloxy-α-[N-[2-(3-carbamoyl-4-hydroxy-phenoxy)-1-methyl-äthyl]-aminomethyl-benzylalkohol als kristalline Masse, die ohne weitere Reinigung hydriert werden kann.

Beispiel 8

Eine Lösung von 26,8 g 5-(2-Oxo-propoxy)-salicylamid und 17,6 g α-(Aminomethyl)-benzylalkohol in 450 ml Methanol wird nach Zusatz von 0,32 g konz. Schwefelsäure und 3 g Platin-auf-Kohle-Katalysator (5%) bis zur Aufnahme von 1 Moläquivalent Wasserstoff unter Normalbedingungen hydriert. Der Katalysator wird abfiltriert, das Lösungsmittel abgedampft und der so erhaltene, rohe α-[N-[2-(3-Carbamoyl-4-hydroxy-phenoxy)-1-methyl-äthyl]-aminomethyl]-benzylalkohol in möglichst wenig Methanol heiss gelöst, die Lösung abgekühlt und mit Kristallen des höher schmelzenden Enantiomeren-Paares angeimpft. Die nach mehrtägigem Stehen erhaltenen Kristalle vom Smp. 160–163° ergeben nach einer weiteren Kristallisation aus Methanol das eine reine Enantiomerenpaar vom Smp. 166–168°. Aus der ersten Mutterlauge wird nach Abdampfen des Lösungsmittels durch fraktionierte Kristallisation unter Verwendung von Aethylacetat bzw. Isopropanol das niedriger schmelzende Enantomeren-Paar vom Smp. 152–154° isoliert.

Das als Ausgangsmaterial benötigte 5-(2-Oxo-propoxy)-salicylamid wird auf folgende Weise hergestellt:

8a) 74 g rohes, gemäss Beispiel 1b) erhaltenes 2,3-Dihydro-2,2-dimethyl-6-(2-oxo-propoxy)-4H-1,3-benzoxazin-4-on werden in einem Gemisch von 150 ml Dioxan und 450 ml 2-n. Salzsäure 45 Minuten auf dem Wasserbad erhitzt. Das Lösungsmittel wird abgedampft und der kristalline Rückstand mit Wasser verrieben und dann abgesaugt. Durch Umkristallisation aus Isopropanol erhält man das 5-(2-Oxo-propoxy)-salicylamid vom Smp. 152–154°.

Beispiel 9
Eine Lösung von 8,0 g R-(-)-α-(Aminomethyl)-benzylalkohol und 12,2 g 5-(2-Oxo-propoxy)-salicylamid in 220 ml Methanol wird analog Beispiel 8 hydriert und aufgearbeitet. Durch Neutralisation der rohen Base mit 5-n. Salzsäure in Methanol erhält man nach Umkristallisation aus Methanol-Äther das Hydrochlorid des R-(-)-α-[N-[2-(3-Carbamoyl-4-hydroxy-phenoxy)-1-methyl-äthyl]-aminomethyl]-benzylalkohols als reines Enantiomeres vom Smp. 180–184° ($[\alpha]_D^{20}$-31±1° (1% in Methanol)).

Aus der Mutterlauge kristallisiert durch Einengen ein Gemisch der Diastereomeren vom Smp. 165–180°.

Beispiel 10
Eine Lösung von 7,2 g 4-Benzyloxy-α-[N-[2-(3-Carbamoyl-4-hydroxy-phenoxy)-äthyl]-aminomethyl]-benzylalkohol in 200 ml Methanol wird in Anwesenheit von Palladium-auf-Kohle-Katalysator (5%) analog Beispiel 7 hydriert. Durch Eindampfen der filtrierten Lösung erhält man rohen α-[N-[2-(3-Carbamoyl-4-hydroxy-phenoxy)-äthyl]-aminomethyl]-4-hydroxybenzylalkohol, der nach Umkristallisation aus Methanol-Isopropanol bei 194–196° schmilzt.

Das Ausgangsmaterial wird auf folgende Weise hergestellt:
a) Ein Gemisch von 21 g α-(Aminomethyl)-4-benzyloxy-benzylalkohol und 12 g 6-(2-Bromäthoxy)-2,3-dihydro-2,2-dimethyl-4H-1,3-benzoxazin-4-on wird 1 Stunde in einem Ölbad von 130° gerührt. Das Reaktionsgemisch wird zwischen 500 ml Äthylacetat und 50 ml 2-n. wässeriger Ammoniaklösung verteilt, die organische Phase abgetrennt, eingedampft und aus Isopropanol umkristallisiert. Man erhält den 4-Benzyloxy-α-[N-[2-(3-Carbamoyl-4-hydroxy-phenoxy)-äthyl]-aminomethyl]-benzylalkohol vom Smp. 160–162°.

Beispiel 11
Eine geschmolzene Mischung von 5,0 g 6-(2-Bromäthoxy)-salicylamid und 13,0 g α-(Aminomethyl)-benzyl-alkohol wird 1 Stunde in einem Bad von 130° gerührt. Das noch warme Reaktionsgemisch wird in 250 ml Aethylacetat gelöst, die Lösung aufeinanderfolgend mit 50 ml gesättigter Kaliumbicarbonat-Lösung, 100 ml Wasser und 100 ml gesättigter wässeriger Natriumchloridlösung gewaschen, über Magnesiumsulfat getrocknet und eingedampft. Der Rückstand wird in 30 ml Aethanol heiss gelöst, woraus beim Abkühlen der α-[N-[2-(2-Carbamoyl-3-hydroxy-phenoxy)-äthyl]-aminomethyl]-benzylalkohol vom Smp. 122–123° auskristallisiert. Die Verbindung bildet ein Hydrochlorid vom Smp. 190–191° (aus Methanol-Isopropanol).

Das Ausgangsmaterial wird wie folgt hergestellt:

a) Ein Gemisch von 23,0 g 2,6-Dihydroxybenzamid, 20,7 g Kaliumcarbonat und 28,2 g 1,2-Dibromäthan wird in 300 ml Acetonitril 2–3 Stunden unter Rückfluss gerührt.

Das Reaktionsgemisch wird noch warm filtriert, das Filtrat eingedampft und der Rückstand aus wenig Methanol umkristallisiert. Man erhält das 6-(2-Bromäthoxy)-salicylamid vom Smp. 120–121°.

Beispiel 12
16,6 g rohes N-[3-(4-Carbamoyl-3-hydroxy-phenoxy)-propyl]-N-(2-hydroxy-2-phenyl-äthyl)-benzylamin wird analog Beispiel 4 hydriert und aufgearbeitet. Man erhält durch Umkristallisation aus Isopropanol den α-[N-[3-(4-Carbamoyl-3-hydroxy-phenoxy)-propyl]-aminomethyl]-benzylalkohol vom Smp. 208–210°.

Das Ausgangsmaterial wird über folgende Stufen hergestellt:

a) Ein Gemisch von 38,6 g 2,3-Dihydro-2,2-dimethyl-7-hydroxy-4H-1,3-benzoxazin-4-on, 55,2 g Kaliumcarbonat, 47,2 g 1-Brom-3-chlor-propan und 600 ml Aceton wird 24 Stunden unter Rückfluss gekocht. Durch Filtration, Einengen des Filtrats auf ⅓ des ursprünglichen Volumens und Abkühlen erhält man das 7-(3-Brompropoxy)-2,3-dihydro-2,2-dimethyl-4H-1,3-benzoxazin-4-on vom Smp. 111–113°.

b) 13,6 g dieser Verbindung werden zusammen mit 32 g Benzylamin unter Rühren während 1½ Stunden auf 100° erhitzt. Aufarbeitung auf Base analog Beispiel 4b, und Abdestillieren des überschüssigen Benzylamin im Hochvakuum ergibt nach Umkristallisation des Rückstands aus Toluol das N-[3-(4-Carbamoyl-3-hydroxy-phenoxy)-propyl]-benzylamin vom Smp. 131–135°.

c) Eine Lösung von 12,7 g N-[3-(4-Carbamoyl-3-hydroxy-phenoxy)-propyl]-benzylamin und 7,6 g Phenyläthylenoxid in 80 ml Isopropanol wird 16 Stunden unter Rückfluss gekocht. Das Reaktionsgemisch wird eingedampft, zwischen je 100 ml 6-n. Salzsäure und Äther verteilt, die wässerige Phase abgetrennt und diese mit 2-n. Ammoniak-Lösung alkalisch gestellt. Das so erhaltene N-[3-(4-Carbamoyl-3-hydroxy-phenoxy)-propyl]-N-(2-hydroxy-2-phenyl-äthyl]-benzylamin wird durch Extraktion mit Aethylacetat isoliert und als Rohprodukt debenzyliert.

Beispiel 13
Eine Lösung von 1,7 g rohem α-[N-[2-(3-Carbomethoxy-4-hydroxy-phenoxy)-1-methyl-äthyl]-aminomethyl]-benzylalkohol in 10 ml Dioxan wird mit 20 ml konz. Ammoniak-Lösung versetzt und 60–70 Stunden bei Raumtemperatur stehen gelassen. Das Reaktionsgemisch wird eingedampft, in 75 ml Äthylacetat gelöst und mit 10 ml gesättigter, wässeriger Natriumchloridlösung gewaschen. Durch Eindampfen der organischen Phase erhält man den rohen α-[N-[2-(3-Carbamoyl-4-hydroxy-phenoxy)-1-methyl-äthyl]-aminomethyl]-benzylalkohol als Diastereoisomerengemisch in Form eines Öles, das aus wenig Äthylacetat kristallisiert wird und mit dem z.B. in Beispiel 1 erhaltenen Produkt identisch ist; Smp. 120–140°.

Das Ausgangsmaterial wird auf folgende Weise erhalten:

a) Eine Lösung von 3,4 g 2,5-Dihydroxybenzoesäuremethylester, 4,0 g Triäthylamin und 2,8 g Chloraceton in 40 ml Acetonitril wird 16 Stunden unter Rückfluss gekocht. Nach Zusatz von 1,4 g Chloraceton und 1,3 g Triäthylamin wird das Reaktionsgemisch weitere 5 Stunden gekocht, dann eingedampft, der Rückstand in 50 ml Toluol gelöst, die Lösung mit 10 ml Wasser, dann mit 10 ml gesättigter wässeriger Natriumchloridlösung gewaschen und über 200 g Silicagel chromatographiert. Die mit Toluol zuerst eluierten Fraktionen enthalten den 5-(2-Oxo-propoxy)-salicylsäuremethylester der aus Isopropanol umkristalliert wird und bei 80–82° schmilzt.

b) Eine Lösung von 0,8 g α-Aminomethyl)-benzylalkohol und 1,2 g 5-(2-Oxopropoxy)-salicylsäuremethylester in 40 ml Methanol wird analog Beispiel 8 hydriert.

Durch Filtrieren und Eindampfen erhält man rohen α-[N-[2-(3-Carbomethoxy-4-hydroxy-phenoxy)-1-methyl-äthyl]-aminomethyl]-benzylalkohol als gelbliches Öl, das roh weiterverarbeitet wird.

Beispiel 14

Eine Lösung von 13,4 g Phenylglyoxal und 19,6 g 5-(2-Aminoäthoxy)-salicylamid in 100 ml Methanol wird 1 Stunde unter Rückfluss erhitzt und dann unter Rühren und Eiskühlung portionenweise mit 7 g Natriumborhydrid versetzt. Das Reaktionsgemisch wird über Nacht bei ca. 20° gerührt, unter Eiskühlung mit 2-n. Salzsäure zersetzt und filtriert. Das Filtrat wird eingeengt, mit konz. Ammoniak-Lösung alkalisch gestellt und mit Äthylacetat extrahiert.

Das durch Eindampfen erhaltene Rohprodukt wird aus Äthanol kristallisiert, wonach man den α[N-[2-(3-Carbamoyl-4-hydroxy-phenoxy)-äthyl]-aminomethyl]-benzylalkohol vom Smp. 174–175° erhält.

14a) Das als Ausgangsmaterial benötigte 5-(2-Aminoäthoxy)-salicylamid vom Smp. 140° erhält man analog Beispiel 21 durch katalytisches Debenzylieren von 5-(2-Benzylaminoäthoxy)-salicylamid mittels 5%igem Palladium-auf-Kohle-Katalysator in Methanol.

Beispiel 15

Analog Beispiel 3 erhält man durch Schmelzen von 5,0 g α-(Aminomethyl)-2-pyridinmethanol und 2,2 g 2,3-Dihydro-2,2-dimethyl-6-(2-bromäthoxy)-4H-1,3-benzoxazin-4-on das α-[N-[2-(3-Carbamoyl-4-hydroxy-phenoxy)-äthyl]-aminomethyl]-2-pyridinmethanol vom Smp. 169–170°, (aus Äthanol-Methanol).

Beispiel 16

Ein Gemisch von 0,96 g Phenyl-äthylenoxid und 2,1 g 4-(2-Amino-propoxy)-salicylamid wird in 25 ml 2-Propanol 15 Stunden unter Rückfluss gekocht. Das Reaktionsgemisch wird hierauf eingedampft, zwischen 10 ml Wasser und 100 ml Äthylacetat verteilt und die organische Phase dreimal mit Wasser gewaschen. Durch Trocknen über Magnesiumsulfat und Eindampfen der organischen Phase erhält man rohen α-[N-[2-(4-Carbamoyl-3-hydroxy-phenoxy)-1-methyl-äthyl]-aminomethyl]-benzylalkohol der analog Beispiel 2 umkristallisiert wird und auf diese Weise die beiden Enantiomeren-Paare vom Smp. 171–173° bzw. 149–151° ergibt.

Das Ausgangsmaterial wird auf folgende Weise hergestellt:

a) Eine Lösung von 25 g 2,3-Dihydro-2,2-dimethyl-7-(2-oxo-propoxy)-4H-1,3-benzoxazin-4-on in 500 ml Methanol wird nach Zusatz von ca. 5 g Raney-Nickel-Katalysator in einen Autoclaven gegeben und anschliessend Ammoniak-Gas aufgepresst, bis der Druck 5 bar beträgt. Hierauf wird bei 50° und 100 bar Wasserstoff-Druck bis zum Stillstand hydriert. Nach Abfiltrieren des Katalysators und Eindampfen der Lösung erhält man das rohe 4-(2-Amino-propoxy)-salicylamid, das nach Umkristallisation aus Methanol bei 185–187° schmilzt.

Beispiel 17

Ein Gemisch von 7,0 g α-(Aminomethyl)-2-furanmethanol und 7,5 g 2,3-Dihydro-2,2-dimethyl-6-(2-bromäthoxy)-4H-1,3-benzoxazin-4-on wird geschmolzen und 1 Stunde in einem Bad von ca. 110° gerührt. Zu dem noch warmen Reaktionsgemisch werden 50 ml Isopropanol und 2 ml. konz. Ammoniak-Lösung gegeben, das Gemisch 5 Minuten gekocht, filtriert und abkühlen gelassen. Nach dem Aufarbeiten erhält man das α-[N-[2-(3-Carbamoyl-4-hydroxy-phenoxy)-äthyl]-aminomethyl]-2-furanmethanol vom Smp. 169–171°.

Beispiel 18

Ein Gemisch von 2,7 g α-[Aminomethyl)-2-thiophenmethanol und 3,6 g 2,3-Dihydro-2,2-dimethyl-6-(2-bromäthoxy)-4H-1,3-benzoxazin-4-on wird zusammen mit 2 ml Triäthylamin 1 Stunde in einem Bad von 100–110° gerührt. Das Reaktionsgemisch wird zwischen 100 ml Ähylacetat und 10 ml 2-n. wässriger Kaliumbicarbonat-Lösung verteilt, die organische Phase abgetrennt, über Magnesiumsulfat getrocknet und eingedampft, wonach man rohes α-[N-[2-(3-Carbamoyl-4-hydroxy-phenoxy)-äthyl]-aminomethyl]-2-thiophenmethanol als kristalline Masse erhält, die nach dem Umkristallisieren aus wenig Isopropanol bei 158–161° schmilzt; nochmaliges Umkristallisieren erhöht den Schmelzpunkt auf 171–172°.

Beispiel 19

Eine Lösung von 14,1 g S-(+)-α-(Aminomethyl)-benzylalkohol und 21,5 g 5-(2-Oxo-propoxy)-salicylamid in 400 ml Methanol wird unter Zusatz von 0,26 g konz. Schwefelsäure über 2 g Platin-auf-Kohle-Katalysator (5%) analog Beispiel 8 hydriert und aufgearbeitet. Der als Schaum anfallende Eindampfrückstand kristallisiert aus Äthylacetat als Diastereomeren-Gemisch vom Smp. 146–161°. Durch fraktionierte Kristallisation aus Isopropanol erhält man ein reines Enantiomeres des S-(+)-α-[N-[2-(3-Carbamoyl-4-hydroxy-phenoxy)-1-methyl-äthyl]-aminomethyl]-benzylalkohols vom Smp. 160–162°. Durch Neutralisation der bei der fraktionierten Kristallisation erhaltenen basischen Mutterlauge mit methanolischer Salzsäure und fraktioniertes Kristallisieren des Hydrochlorids aus Methanol erhält man das andere Enantiomere als Hydrochlorid vom Smp. 181–183°. Beide Enantiomere als Hydrochloride zeigen die gleiche spezifische Drehung $[\alpha]_D^{20°}$ = + 33°±1° (C = 1, Methanol), unterscheiden sich jedoch in ihren C-NMR-Spektren.

Beispiel 20

Eine Lösung von 8,7 g 4-Benzyloxy-α-[N-[2-(4-Carbamoyl-3-hydroxy-phenoxy)-äthyl]-aminomethyl]-benzylalkohol in 100 ml Dioxan wird unter Zusatz von 2 g Palladium-auf-Kohle-Katalysator (5%) unter Normalbedingungen bis zur Aufnahme von 1 Äquivalent Wasserstoff hydriert. Das ausgefallene Produkt wird nach Zusatz von 200 ml Methanol durch Erwärmen in Lösung gebracht, der Katalysator abfiltriert und das Filtrat eingedampft. Durch Umkristallisation auf Isopropanol erhält man kristallinen α-[N-[2-(4-Carbamoyl-3-hydroxy-phenoxy)-äthyl]-aminomethyl]-4-hydroxy-benzylalkohol vom Smp. 181–182°.

20a) Das Ausgangsmaterial kann man analog Beispiel 10a) unter Verwendung von 7-(2-Bromäthoxy)-2,3-dihydro-2,2-dimethyl-4H-1,3-benzoxazin-4-on herstellen. Der so erhaltene 4-Benzyloxy-α-[N-[2-(4-Carbamoyl-3-hydroxy-phenoxy)-äthyl]-aminomethyl]-benzylalkohol schmilzt bei 181–183°.

Beispiel 21

Eine Lösung von 45 g rohem α-[N-Benzyl-N-[2-(3-carbamoyl-4-hydroxy-phenoxy)-äthyl]-aminomethyl]-benzylalkohol in 500 ml Methanol wird unter Zusatz von 5 g Palladium-auf-Kohle-Katalysator (5%) analog Beispiel 4 hydriert und aufgearbeitet. Man erhält α-[N-[2-(3-Carbamoyl-4-hydroxy-phenoxy)-äthyl]-aminomethyl]-benzylalkohol vom Smp. 174–175° (aus Äthanol). Das Methansulfonat schmilzt bei 182–183° (aus Methanol).

Das Ausgangsmaterial wird auf folgende Weise hergestellt:

21a) Eine Suspension von 60 g 6-(2-Bromäthoxy)-2,3-dihydro-2,2-dimethyl-4H-1,3-benzoxazin-4-on in 110 ml Benzylamin wird unter Rühren während 30 Minuten in einem Bad von 80° erwärmt. Das Reaktionsgemisch wird hierauf unter Eiskühlung mit konz. Salzsäure auf ca. pH 3 eingestellt und zur Kristallisation stehengelassen. Nach 4 Stunden werden die Kristalle abgesaugt, mit je 10 ml Wasser und Äthylacetat gewaschen und getrocknet. Das so erhaltene rohe 5-(2-Benzylamino-äthoxy)-salicylamid-hydrochlorid schmilzt bei 214–216°. Die daraus freigesetzte Base schmilzt bei 107–108° (aus Äthylacetat-Äther).

21b) Eine Lösung von 13,2 g Phenyläthylenoxid und 28,6 g 5-(2-Benzylamino)-äthoxy)-salicylamid in 100 ml Isopropanol wird während 24 Stunden unter Rückfluss erhitzt. Durch Abdampfen des Lösungsmittels erhält man rohen α-[N-Benzyl-N-[2-(3-carbamoyl-4-hydroxy-phenoxy)-äthyl]-aminomethyl]-benzylalkohol als Öl, welches ohne weitere Reinigung zur Debenzylierung eingesetzt werden kann.

Beispiel 22

Ein Gemisch von 13,7 g α-(Aminomethyl)-benzylalkohol und 9,4 g 6-(3-Brompropoxy)-2,3-dihydro-2,2-dimethyl-4H-1,3-benzoxazin-4-on wird während 45 Minuten unter Rühren in einem Bad von 120° erhitzt. Das Reaktionsgemisch wird abgekühlt, mit 250 ml Äthylacetat versetzt und die Lösung 2mal nacheinander mit je 50 ml wässriger, gesättigter Kaliumbicarbonat-Lösung, Wasser und gesättigter Kochsalz-Lösung gewaschen, über Magnesiumsulfat getrocknet und eingedampft. Der ölige Rückstand wird in wenig Isopropanol gelöst und mit einer Lösung von Salzsäuregas in Methanol (~-5-n.) neutralisiert. Man erhält so den α-[N-[3-(3-Carbamoyl-4-hydroxy-phenoxy)-propyl]-aminomethyl]-benzylalkohol als Hydrochlorid vom Smp. 189–192°.

Das Ausgangsmaterial wird auf folgende Weise hergestellt:

22a) Eine Mischung von 9,7 g 2,3-Dihydro-2,2-dimethyl-6-hydroxy-4H-1,3-benzoxazin-4-on, 41 g 1,3-Dibrom-propan und 10,4 g Kaliumcarbonat wird 2½ Stunden unter Rückfluss gerührt. Das Reaktionsgemisch wird mit Toluol verdünnt, filtriert und das Filtrat eingedampft. Zum Rückstand werden 20 ml Äther gegeben und vom ausgefällten Schlamm abfiltriert. Nochmaliges Eindampfen und Verreiben mit 50 ml Petroläther ergeben 14 g rohes 6-(3-Brom-propoxy)-2,3-dihydro-2,2-dimethyl-4H-1,3-benzoxazin-4-on als kristalline Masse. Nach Umkristallisation aus wenig Petroläther schmilzt das Produkt bei 115–118°.

Beispiel 23

Eine Lösung von 13,9 g rohem α-[N-Benzyl-N-[3-(3-carbamoyl-4-hydroxy-phenyl)-propyl]-aminomethyl]-benzylalkohol in 150 ml Methanol wird in Gegenwart von 1,4 g Palladium-auf-Kohle-Katalysator (5%) unter Normalbedingungen bis zum Stillstand der Wasserstoffaufnahme hydriert. Durch Filtration der Lösung, Eindampfen und Verreiben mit Äther erhält man einen Feststoff, der nach Umkristallisation aus wenig Isopropanol

den α-[N-[3-(3-Carbamoyl-4-hydroxy-phenyl)-propyl]-aminomethyl]-benzylalkohol vom Smp. 151–153° darstellt.

Die Ausgangsstoffe können auf die nachfolgend beschriebene Weise erhalten werden:

23a) 3-(4-Hydroxyphenyl)-propionsäure wird über das mit Chlorameisensäuremethylester erhaltene gemischte Anhydrid mit Benzylamin in das 3-(4-Hydroxyphenyl)-propionsäure-N-benzylamid vom Smp. 115–116° übergeführt.

23b) Aus dem Natriumsalz des 3-(4-Hydroxyphenyl)-propionsäure-N-benzylamid und Kohlendioxid wird bei einer Temperatur von 180° während 4 Stunden und einem Druck von 55 bar unter den Bedingungen der Kolbe-Synthese das 3-(3-Carboxy-4-hydroxyphenyl)-propionsäure-N-benzylamid vom Smp. 180–181° erhalten.

23c) Durch Veresterung von 3-(3-Carboxy-4-hydroxyphenyl)-propionsäure-N-benzylamid mit einem Gemisch von Methanol und konz. Schwefelsäure während 48-stündigem Kochen unter Rückfluss wird 3-(3-Methoxycarbonyl-4-hydroxyphenyl)-propionsäure-N-benzylamid vom Smp. 139–140° (aus Äthylacetat) erhalten.

23d) Umsetzung von 3-(3-Methoxycarbonyl-4-hydroxyphenyl)-pripionsäure-N-benzylamid mit Benzylbromid in Gegenwart von Kaliumcarbonat in Aceton als Lösungsmittel während 15-stündigem Kochen unter Rückfluss ergibt das 3-(4-Benzyloxy-3-methoxycarbonyl-phenyl)-propionsäure-N-benzylamid als gelbliches Öl.

23e) Durch selektive Reduktion der Amidgruppe im 3-(4-Benzyloxy-3-methoxycarbonyl-phenyl)-propionsäure-N-benzylamid mittels Diboran in Tetrahydrofuran während 48 Stunden bei einer Temperatur von 20–25° wird das N-[3-(4-Benzyloxy-3-methoxycarbonyl-phenyl)-propyl]-benzylamin erhalten, welches als Rohprodukt mittels Wasserstoff in Gegenwart von Palladium-auf-Kohle-Katalysator (50%) in Methanol als Lösungsmittel bei einer Temperatur von 15–20° in das N-[3-(4-Hydroxy-3-methoxycarbonyl-phenyl)-propyl]-benzylamid vom Smp. 75–77° (aus Isopropanol) übergeführt wird.

23f) Eine Lösung von 27 g N-[3-(4-Hydroxy-3-methoxy-carbonyl-phenyl)-propyl]-benzylamin in 100 ml Dioxan wird mit 200 ml konz. Ammoniak verestert und 3–4 Tage bei 20–30° stehengelassen. Das Reaktionsgemisch wird eingedampft, zwischen Wasser und Äthylacetat verteilt und die organische Phase abgetrennt. Übliche Aufarbeitung ergibt 14,5 g rohes N-[3-Carbamoyl-4-hydroxy-phenyl)-propyl]-benzylamin als Öl, welches ohne weitere Reinigung weiterverarbeitet wird.

23g) Eine Lösung von 8,5 g rohem N-[3-(3-Carbamoyl-4-hydroxyphenyl)-propyl]-benzylamin und 10 g Phenyläthylenoxid in 100 ml Isopropanol wird 8 Stunden unter Rückfluss gekocht und dann eingedampft. Der ölige Rückstand wird durch Verrühren mit Petroläther von überschüssigem Phenyläthylenoxid befreit. Der in Petroläther schwer lösliche rohe, ölige α-[N-Benzyl-N-[3-(3-carbamoyl-4-hydroxyphenyl)-propyl]-aminomethyl]-benzylalkohol wird ohne weitere Reinigung als Rohprodukt weiterverarbeitet.

Beispiel 24

Eine Lösung von 31 g rohem N-[2-(3-Carbamoyl-4-hydroxy-phenoxy)-äthyl]-N-[2-(3,5-dibenzyloxyphenyl)-2-hydroxy-äthyl]-benzylamin in 300 ml Methanol wird unter Normalbedingungen über 6 g Palladium-auf-Kohle-Katalysator (5%) bis zur Aufnahme von 3 Moläquivalenten Wasserstoff hydriert. Der Katalysator wird abfiltriert, das Filtrat eingedampft und der Rückstand in 200 ml Isopropanol gelöst. Filtration über Kieselgel und Eindampfen des Filtrates ergeben den α-[N-[2-(3-Carbamoyl-4-hydroxy-phenoxy)-äthyl]-aminomethyl]-3,5-dihydroxy-benzylalkohol als bräunlichen Schaum. Die Verbindung wird durch folgende Signale in Protonenkernresonanzspektrum in Hexadeuterodimethylsulfoxid als Lösungsmittel charakterisiert:

Chemische Verschiebung:
2,74 (d, 2H); 3,0 (t,2H); 4,05 (t, 2H); 4,5 (t,1H); 6,1 (d,1H); 6,24 (d,2H); 6,8 (d,1H); 7,05 (g,1H); 7,47 (d,1H).

Der Ausgangsstoff kann auf folgende Weise hergestellt werden:

24a) Eine Lösung von 20,6 g ω-Brom-3,5-dibenzyloxyacetophenon in 300 ml Aceton wird portionenweise unter Rühren mit 28,6 g 5-(2-Benzylamino-äthoxy)-salicylamid versetzt und das Reaktionsgemisch über Nacht bei Raumtemperatur gerührt. Die ausgefallenen Kristalle werden abgesaugt, und das Filtrat eingedampft, wonach man rohes ω-[N-[2-(3-Carbamoyl-4-hydroxy-phenoxy)-äthoxy-äthyl]-benzylamino-3,5-dibenzyloxy-acetophenon erhält.

24b) Eine Lösung von 39 g des erhaltenen Rohproduktes in einem Gemisch von je 200 ml Äthanol und Methanol wird im Abstand von 2–3 Stunden viermal mit je 3 g Natriumborhydrid versetzt und anschliessend über Nacht gerührt. Unter Eiskühlung wird das Gemisch durch Eintropfen von 2-n. Salzsäure auf pH 4 eingestellt und anschliessend eingedampft. Der Eindampfrückstand wird mit 200 ml Wasser versetzt, mit konz. Ammoniak-Lösung alkalisch gestellt und mit 400 ml Äthylacetat extrahiert. Die organische Phase wird abgetrennt, über Magnesiumsulfat getrocknet und eingedampft, wonach man N-[2-(3-Carbamoyl-4-hydroxy-phenoxy)-äthyl]-N-[2-(3,5-dibenzyloxyphenyl)-2-hydroxy-äthyl]-benzylamin als dickflüssiges Rohprodukt erhält, welches als solches weiterverarbeitet wird.

Beispiel 25

Eine Suspension von 21,9 g 5-(2-Aminoäthoxy)-salicylamid, 36,0 α-(Brommethyl)-benzylalkohol und 30 g Kaliumbicarbonat in 240 ml Äthanol wird während 15 Stunden unter Rühren zum Rückfluss erhitzt. Danach wird das Reaktionsgemisch abgekühlt, filtriert und eingedampft. Der Eindampfrückstand wird zwischen 300 ml Äthylacetat und

100 ml Wasser verteilt, die organische Phase abgetrennt, über Magnesiumsulfat getrocknet und eingedampft. Der Rückstand wird in möglichst wenig Äthanol heiss gelöst und zur Kristallisation gebracht, wonach man den α-[N-[2-(3-Carbamoyl-4-hydroxy-phenoxy)-äthyl]-aminomethyl]-benzylalkohol vom Smp. 174–175° (aus Äthanol) erhält.

Beispiel 26
Eine Suspension von 14,5 g Phenyläthylenoxid und 19,6 g des nach Beispiel 14a) erhaltenen 5-(2-Aminoäthoxy)-salicylamid in 50 ml Isopropanol wird 30 Minuten unter Rückfluss gekocht. Nach dem Abkühlen kristallisiert allmählich der α-[N-[2-(3-Carbamoyl-4-hydroxy-phenoxy)-äthyl]-aminomethyl]-benzylalkohol aus, der nach Umkristallisation aus Äthanol bei 174–175° schmilzt.

Beispiel 27
Tabletten enthaltend 20 mg an aktiver Substanz werden in folgender Zusammensetzung in üblicher Weise hergestellt:

Zusammensetzung:

| | |
|---|---|
| α-[N-[2-(3-Carbamoyl-4-hydroxy-phenoxy)-1-methyl-äthyl]-aminomethyl]-benzylalkohol vom Smp. 152–154° | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:
α-[N-[2-(3-Carbamoyl-4-hydroxy-phenoxy)-1-methyl-äthyl]-aminomethyl]-benzylalkohol vom Smp 152–154° wird mit einem Teil der Weizenstärke, mit Milchzucker und kolloidaler Kieselsäure gemischt und die Mischung durch ein Sieb gerieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 28
Kapseln enthaltend 20 mg an aktiver Substanz werden wie folgt auf übliche Weise hergestellt:

| | |
|---|---|
| α-[N-[2-(3-Carbamoyl-4-hydroxy-phenoxy)-1-methyl-äthyl]-aminomethyl]-benzylalkohol vom Smp. 152–154° | 2500 mg |
| Talkum | 80 mg |
| Kolloidale Kieselsäure | 20 mg |

Herstellung:
Die aktive Substanz wird mit Talkum und kolloidaler Kieselsäure innig gemischt, das Gemisch durch ein Sieb mit 0,5 mm Maschenweite getrieben und dieses in Portionen von jeweils 21 mg in Hartgelatinekapseln geeigneter Grösse abgefüllt.

Beispiel 29
Ein Sirup enthaltend 2 Gew.%/Volumen an aktiver Substanz wird wie folgt auf übliche Weise hergestellt:

Zusammensetzung:

| | |
|---|---|
| α-[N-[2-(3-Carbamoyl-4-hydroxy-phenoxy)-äthyl]-aminomethyl-benzylalkohol vom Smp. 152–154° | 2,0 g |
| Saccharin | 0,6 g |
| Zucker | 30,0 g |
| Glycerin | 5,0 g |
| Geschmacksstoffe | 0,1 g |
| Äthanol (86%) | 10,0 ml |
| dest. $H_2O$ | ad 100 ml |

Herstellung:
Der Zucker, Saccharin und Glycerin werden in 60 g Wasser gelöst. Hierzu gibt man unter Rühren die Lösung des Wirkstoffes und des Geschmacksstoffes in Äthanol. Danach wird die Mischung mit dest. Wasser auf 100 ml aufgefüllt.

Beispiel 30
Anstelle der in den Beispielen 27 bis 29 als aktive Substanz verwendeten Verbindungen können auch folgende Verbindungen der Formel I, oder deren pharmazeutisch annehmbare nicht-toxischen Säureadditionssalze als Wirkstoffe in Tabletten, Dragées, Kapseln etc. verwendet werden: α-[N-[2-(4-Carbamoyl-3-hydroxy-phenoxy)-1-methyl-äthyl]-aminomethyl]-benzylalkohol als Enantiomerenpaar vom Smp. 171–173° als auch in Form des Enantiomerenpaars vom Smp. 149–151°;
α-[N-[2-(4-Carbamoyl-3-hydroxy-phenoxy)-äthyl]-aminomethyl]-benzylalkohol,
α-[N-[2-(3-Carbamoyl-4-hydroxy-phenoxy)-1-methyl-äthyl]-aminomethyl]-3-pyridinmethanol,
α-[N-[2-(3-Carbamoyl-4-hydroxy-phenoxy)-1-methyläthyl]-aminomethyl]-4-hydroxybenzylalkohol,
R-(-)-α-[N-[2-(3-Carbamoyl-4-hydroxy-phenoxy)-1-methyläthyl]-aminomethyl]-benzylalkohol,
α-[N-[2-(3-Carbamoyl-4-hydroxy-phenoxy)-äthyl]-aminomethyl]-4-hydroxybenzylalkohol,
α-[N-[2-(2-Carbamoyl-3-hydroxy-phenoxy)-äthyl]-aminomethyl]-benzylalkohol,
α-[N-[3-(4-Carbamoyl-3-hydroxy-phenoxy)-propyl]-aminomethyl]-benzylalkohol,
α-[N-[2-(3-Carbamoyl-4-hydroxy-phenoxy)-äthyl]-aminomethyl]-benzylalkohol,
α-[N-[2-(3-Carbamoyl-4-hydroxy-phenoxy)-äthyl]-aminomethyl-2-pyridinmethanol,
α-[N-[3-(3-Carbamoyl-4-hydroxy-phenoxy)-propyl]-aminomethyl]-benzylalkohol,
α-[N-[2-(3-Carbamoyl-4-hydroxy-phenoxy)-äthyl]-aminomethyl]-2-furanmethanol,

α-[N-[2-(3-Carbamoyl-4-hydroxy-phenoxy)-äthyl]-aminomethyl]-2-thiophenmethanol, Enantiomeres des S(+)-α-[N-[2-(3-Carbamoyl-4-hydroxy-phenoxy)-1-methyl-äthyl]-aminomethyl]-benzylalkohol vom Smp. 160–162° (Smp. des Hydrochlorids 189–191°) als auch das Enantiomere vom Smp. des Hydrochlorids 181–183°, α-[N-[2-(4-Carbamoyl-3-hydroxy-phenoxy)-äthyl]-aminomethyl]-4-hydroxy-benzylalkohol, α-[N-[3-(3-Carbamoyl-4-hydroxy-phenoxy)-propyl]-aminomethyl]-benzylalkohol, α-[N-[3-(3-Carbamoyl-4-hydroxy-phenoxy)-propyl]-aminomethyl]-benzylalkohol oder α-[N-[2-(3-Carbamoyl-4-hydroxy-phenoxy]-äthyl]-aminomethyl]-3,5-dihydroxybenzylalkohol.

**Patentansprüche für die Vertragsstaaten BE, CH, DE, FR, GB, IT, LU, NL, SE**

1. N-alkylierte Aminoalkohole der Formel

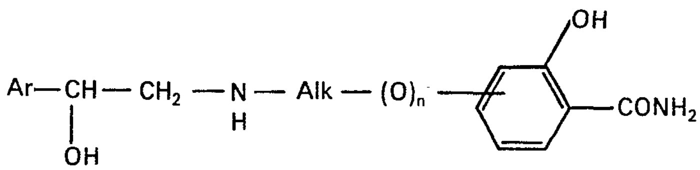
(I)

worin Ar einen unsubstituierten oder durch Hydroxy substituierten Rest aromatischen Charakters, n die Werte Null oder 1, Alk einen Alkylenrest mit 2 bis 5 Kohlenstoffatomen bedeutet, wobei das Stickstoffatom und das Sauerstoffatom, oder, falls n für Null steht, der Phenylrest durch mindestens 2 Kohlenstoffatome voneinander getrennt sind als Racematgemische, stereoisomere (diastereomere) Racemate oder optische Antipoden.

2. Verbindungen der Formel I, worin Ar jeweils unsubstituiertes oder durch 1 oder 2 Hydroxygruppen substituiertes Phenyl, 2- oder 3- oder 4-Pyridyl, 3- oder 4- oder 6-Pyridazinyl, 2- oder 4- oder 5-Pyrimidinyl oder 2-Pyrazinyl, 2- oder 3-Furyl, 2- oder 3-Pyrryl, 2- oder 3-Thienyl, 2- oder 4-Oxazolyl, 2- oder 4-Thiazolyl, 3- oder 5-Pyrazolyl, 2- oder 4-Imidazolyl, 1,2,4-Thiadiazol-3- oder 5-yl oder 1,3,4-Thiadiazol-2-yl und Alk einen Alkylenrest mit 2 bis 4 Kohlenstoffatomen bedeutet, wobei das Stickstoffatom und das Sauerstoffatom, oder, falls n für Null steht, der Phenylrest durch 2 bis 3 Kohlenstoffatome voneinander getrennt sind, und n für Null oder 1 steht.

3. Verbindungen der Formel I, worin Ar jeweils unsubstituiertes oder durch 1 oder 2 Hydroxygruppen substituiertes Phenyl, 2- oder 3-Pyridyl, 2- oder 3-Furyl oder 2- oder 3-Thienyl und Alk einen Alkylenrest mit 2 bis 3 Kohlenstoffatomen bedeutet, wobei das Stickstoffatom und das Sauerstoffatom, oder, falls n für Null steht, der Phenylrest, durch 2 bis 3 Kohlenstoffatome voneinander getrennt sind, und n für Null oder 1 steht.

4. Verbindungen der Formel I, worin Ar jeweils unsubstituiertes oder durch 1 oder 2 Hydroxygruppen substituiertes Phenyl, 2- oder 3-Pyridyl, 2-Furyl oder 2-Thienyl und Alk einen Alkylenrest mit 2 bis 3 Kohlenstoffatomen bedeutet, wobei das Stickstoffatom und das Sauerstoffatom, oder, falls n für Null steht, der Phenylrest, durch 2 bis 3 Kohlenstoffatome voneinander getrennt sind, und n für Null oder 1 steht.

5. α-[N-[2-(3-Carbamoyl-4-hydroxy-phenoxy)-1-methyl-äthyl]-aminomethyl]-benzylalkohol als Enantiomerenpaar vom Smp. 166–168°.

6. α-[N-[2-(3-Carbamoyl-4-hydroxy-phenoxy)-1-methyl-äthyl]-aminomethyl]-benzylalkohol als Enantiomerenpaar vom Smp. 152–154°.

7. α-[N-[2-(3-Carbamoyl-4-hydroxy-phenoxy)-äthyl]-aminomethyl]-benzylalkohol.

8. α-[N-[2-(4-Carbamoyl-3-hydroxy-phenoxy)-äthyl]-aminomethyl]-4-hydroxy-benzylalkohol.

9. Salze von Verbindungen der Ansprüche 1 bis 8.

10. Pharmazeutisch annehmbare, nichttoxische Säureadditionssalze von Verbindungen der Ansprüche 1 bis 8.

11. Pharmazeutische Präparate enthaltend eine Verbindung der Ansprüche 1 bis 8 und 10 zusammen mit inerten Hilfs- und Trägerstoffen.

12. Verbindungen der Formel I als Arzneimittel.

13. Verbindungen der Formel I als Stimulatoren cardialer β-Rezeptoren.

14. Verwendung von Verbindungen der Formel I zur Herstellung von Arzneimitteln.

15. Verbindungen der Formel I zur Verwendung bei der Bekämpfung der Herzinsuffizienz.

16. Verfahren zur Herstellung von N-alkylierten Aminoalkoholen der Formel

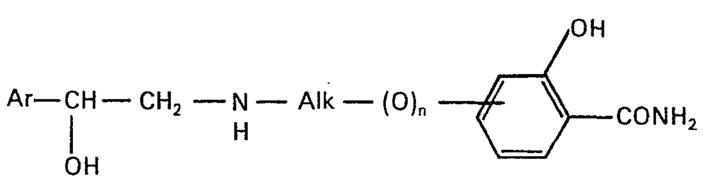
(I)

worin Ar einen unsubstituierten oder durch Hydroxy substituierten Rest aromatischen Charakters, n die Werte Null oder 1 und Alk einen Alkylenrest mit 2 bis 5 Kohlenstoffatomen bedeuten, wobei das Stickstoffatom und das Sauerstoffatom, oder, falls n für Null steht, der Phenylrest

durch mindestens 2 Kohlenstoffatome voneinander getrennt sind, und deren Salze, dadurch gekennzeichnet, dass man

a) eine Verbindung der Formel

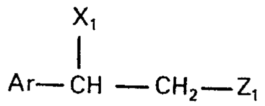

(II)

mit einer Verbindung der Formel

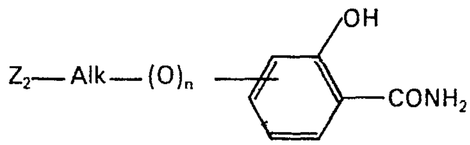

(III),

worin eine der Gruppen $Z_1$ und $Z_2$ eine reaktionsfähige veresterte Hydroxygruppe darstellt und die andere für die primäre Aminogruppe steht und $X_1$ Hydroxy bedeutet, oder worin $X_1$ und $Z_1$ zusammen die Epoxygruppe bedeuten und $Z_2$ für die primäre Aminogruppe steht, und Ar, Alk und n obige Bedeutung haben, umsetzt, oder

b) in einer Verbindung der Formel

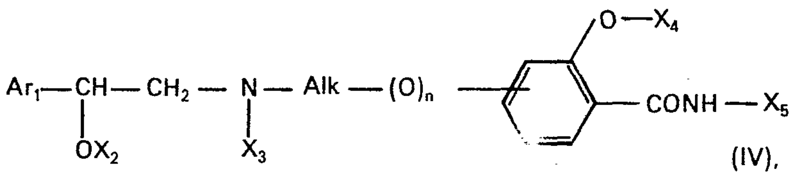

(IV),

worin $Ar_1$ die Bedeutung von Ar hat oder einen Rest Ar bedeutet, der durch 1 bis 2 in Hydroxy überführbare Gruppen substituiert ist, $X_2$, $X_3$ und $X_4$ jeweils Wasserstoff oder einen durch Wasserstoff ersetzbaren Substituenten bedeuten, und $X_5$ für Wasserstoff steht, oder $X_2$ und $X_3$ und/oder $X_4$ und $X_5$ zusammen einen zweiwertigen, durch zwei Wasserstoffatome ersetzbaren Rest bedeuten mit der Massgabe, dass mindestens einer der Reste $X_2$, $X_3$ und $X_4$ von Wasserstoff verschieden ist, oder mindestens $Ar_1$ einen Rest Ar bedeutet, der durch 1 bis 2 in Hydroxy überführbare Gruppen substituiert ist, oder mindestens $X_2$ und $X_3$ zusammen oder $X_4$ und $X_5$ zusammen einen zweiwertigen, durch zwei Wasserstoffatome ersetzbaren Rest darstellen, oder in einem Salz davon, das von Wasserstoff verschiedene $X_2$, $X_3$, $X_4$ oder $X_4$ und $X_5$ zusammen durch Wasserstoff ersetzt, und/oder in einem Rest $Ar_1$ vorhandenes substituiertes Hydroxy in freies Hydroxy überführt, oder

c) in einer Verbindung der Formel

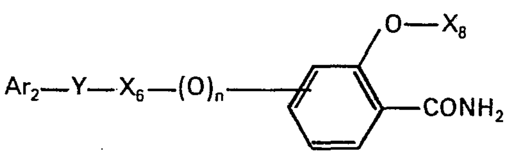

(V),

worin $X_6$ eine reduzierbare Gruppe der Formeln $-CH=N-Alk$ (Va) bzw. $-CH_2-N=Alk_1-$ (Vb) oder $-C(=X_7)-N(X_8)-Alk$ (Vc), bzw. $-CH_2-N(X_8)-C(=X_7)-Alk_2$ (Vd) oder eine Gruppe $-CH_2-N(X_8)-Alk$ (Ve) ist, wobei $Alk_1$ für den einem Rest Alk entsprechenden Alkyl-ylidenrest steht, und $Alk_2$ einem um eine an das Stickstoffatom gebundene Methylengruppe verkürzten Rest Alk entspricht, $X_7$ den Oxo- oder Thioxorest und $X_8$ Wasserstoff oder einen unter den Bedingungen für die Reduktion von $X_6$ und/oder Y durch Wasserstoff ersetzbaren Rest darstellt, und Y für einen Rest der Formel -CO- (Vf) oder $-CH(OX_8)-$ (Vg) steht, in der $X_8$ die vorstehend angegebene Bedeutung hat, $Ar_2$ einem Rest Ar entspricht, jedoch gegebenenfalls anstelle von einem oder zwei Hydroxy eine oder zwei Gruppen $OX_8$, in welcher $X_8$ die vorstehend angegebene Bedeutung hat, trägt und n Null oder 1 bedeutet, wobei stets $X_6$ eine reduzierbare Gruppe Va bis Vd und/oder Y eine Carbonylgruppe Vf ist, diese Gruppe(n) reduziert und im gleichen Arbeitsgang die von Wasserstoff verschiedenen Gruppen $X_8$ durch Wasserstoff ersetzt, oder

d) eine Verbindung der Formel

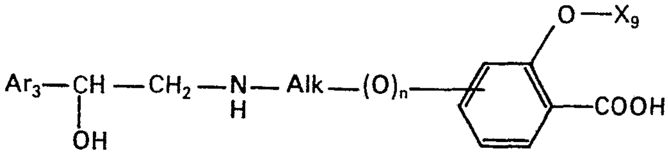

(VI)

worin $Ar_3$ die Bedeutung von Ar hat oder einen Rest Ar darstellt, der durch 1 bis 2 mittels Ammonolyse in Hydroxy überführbare Gruppen substituiert ist, $X_9$ Wasserstoff oder eine mittels Ammonolyse abspaltbare Gruppe bedeutet, oder ein reaktionsfähiges Derivat einer der in Formel (VI) definierten Carbonsäuren mit Ammoniak umsetzt und gleichzeitig gegebenenfalls vorhandene Reste $X_9$ abspaltet und durch Wasserstoff ersetzt, und/oder wenn erwünscht, eine erhaltene freie Verbindung in ein Salz oder ein erhaltenes Salz in die freie Verbindung überführt, und/oder wenn erwünscht, ein erhaltenes Racematgemisch in die beiden stereoisomeren (diastereomeren) Racemate oder ein erhaltenes Racemat in die optischen Antipoden auftrennt.

17. Verfahren nach Anspruch 16, dadurch gekennzeichnet, dass man eine erhaltene Verbindung der Formel I in ein pharmazeutisch annehmbares, nicht-toxisches Säureadditionssalz überführt.

**Patentansprüche für den Vertragsstaat AP**

1. Verfahren zur Herstellung von N-alkylierten Aminoalkoholen der Formel

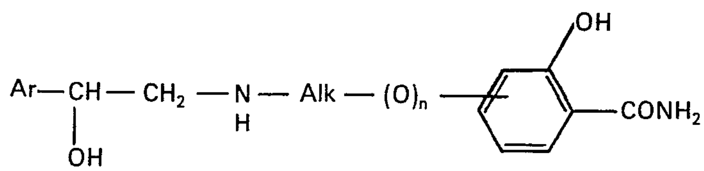 (I)

worin Ar einen unsubstituierten oder durch Hydroxy substituierten Rest aromatischen Charakters, n die Werte Null oder 1 und Alk einen Alkylenrest mit 2 bis 5 Kohlenstoffatomen bedeuten, wobei das Stickstoffatom und das Sauerstoffatom, oder, falls n für Null steht, der Phenylrest durch mindestens 2 Kohlenstoffatome voneinander getrennt sind, und deren Salze, dadurch gekennzeichnet, dass man

a) eine Verbindung der Formel

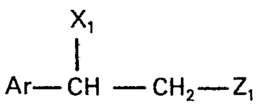 (II)

mit einer Verbindung der Formel

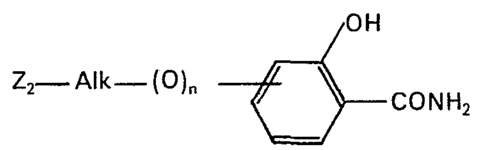 (III)

worin eine der Gruppen $Z_1$ und $Z_2$ eine reaktionsfähige veresterte Hydroxygruppe darstellt und die andere für die primäre Aminogruppe steht und $X_1$ Hydroxy bedeutet, oder worin $X_1$ und $Z_1$ zusammen die Epoxygruppe bedeuten und $Z_2$ für die primäre Aminogruppe steht, und Ar, Alk und n obige Bedeutung haben, umsetzt, oder

b) in einer Verbindung der Formel

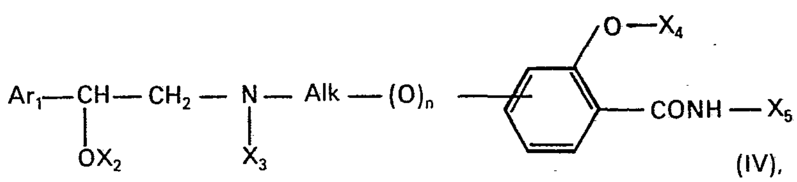 (IV),

worin $Ar_1$ die Bedeutung von Ar hat oder einen Rest Ar bedeutet, der durch 1 bis 2 in Hydroxy überführbare Gruppen substituiert ist, $X_2$, $X_3$ und $X_4$ jeweils Wasserstoff oder einen durch Wasserstoff ersetzbaren Substituenten bedeuten, und $X_5$ für Wasserstoff steht, oder $X_2$ und $X_3$ und/oder $X_4$ und $X_5$ zusammen einen zweiwertigen, durch zwei Wasserstoffatome ersetzbaren Rest bedeuten mit der Massgabe, dass mindestens einer der Reste $X_2$, $X_3$ und $X_4$ von Wasserstoff verschieden ist, oder mindestens $Ar_1$ einen Rest Ar bedeutet, der durch 1 bis 2 in Hydroxy überführbare Gruppen substituiert ist, oder mindestens $X_2$ und $X_3$ zusammen oder $X_4$ und $X_5$ zusammen einen zweiwertigen, durch zwei Wasserstoffatome ersetzbaren Rest darstellen, oder in einem Salz davon, das von Wasserstoff verschiedene $X_2$, $X_3$, $X_4$ oder $X_4$ und $X_5$ zusammen durch Wasserstoff ersetzt, und/oder in einem Rest $Ar_1$ vorhandenes substituiertes Hydroxy in freies Hydroxy überführt, oder

c) in einer Verbindung der Formel

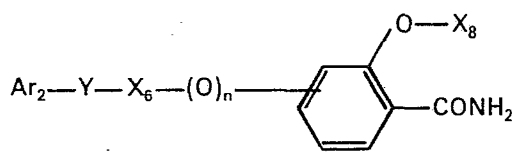 (V),

worin $X_6$ eine reduzierbare Gruppe der Formeln $-CH=N-Alk$ (Va) bzw. $-CH_2-N=Alk_1-$ (Vb) oder $-C(=X_7)-N(X_8)-Alk$ (Vc), bzw. $-CH_2-N(X_8)-C(=X_7)-Alk_2$ (Vd) oder eine Gruppe $-CH_2-N(X_8)-Alk$ (Ve) ist, wobei $Alk_1$ für den einem Rest Alk entsprechenden Alkyl-ylidenrest steht, und $Alk_2$ einem um eine an das Stickstoffatom gebundene Methylengruppe verkürzten Rest Alk entspricht, $X_7$ den Oxo- oder Thioxorest und $X_8$ Wasserstoff oder einen unter den Bedingungen für die Reduktion von $X_6$ und/oder Y durch Wasserstoff ersetzbaren Rest darstellt, und Y für einen Rest der Formel -CO- (Vf) oder $-CH(OX_8)-$ (Vg) steht, in der $X_8$

die vorstehend angegebene Bedeutung hat, $Ar_2$ einem Rest Ar entspricht, jedoch gegebenenfalls anstelle von einem oder zwei Hydroxy eine oder zwei Gruppen $OX_8$, in welcher $X_8$ die vorstehend angegebene Bedeutung hat, trägt und n Null oder 1 bedeutet, wobei stets $X_6$ eine reduzierbare Gruppe Va bis Vd und/oder Y eine Carbonylgruppe Vf ist, diese Gruppe(n) reduziert und im gleichen Arbeitsgang die von Wasserstoff verschiedenen Gruppen $X_8$ durch Wasserstoff ersetzt, oder

d) eine Verbindung der Formel

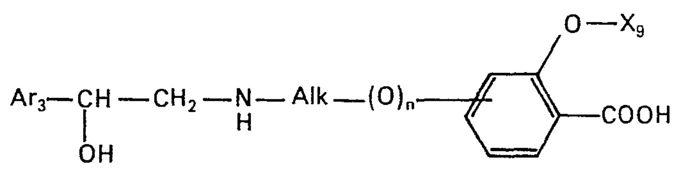

(VI)

worin $Ar_3$ die Bedeutung von Ar hat oder einen Rest Ar darstellt, der durch 1 bis 2 mittels Ammonolyse in Hydroxy überführbare Gruppen substituiert ist, $X_9$ Wasserstoff oder eine mittels Ammonolyse abspaltbare Gruppe bedeutet, oder ein reaktionsfähiges Derivat einer der in Formel (VI) definierten Carbonsäuren mit Ammoniak umsetzt und gleichzeitig gegebenenfalls vorhandene Reste $X_9$ abspaltet und durch Wasserstoff ersetzt, und/oder wenn erwünscht, eine erhaltene freie Verbindung in ein Salz oder ein erhaltenes Salz in die freie Verbindung überführt, und/oder wenn erwünscht, ein erhaltenes Racematgemisch in die beiden stereoisomeren (diastereomeren) Racemate oder ein erhaltenes Racemat in die optischen Antipoden auftrennt.

2. Verfahren zur Herstellung von α-[N-[2-(3-Carbamoyl-4-hydroxy-phenoxy)-1-methyl-äthyl]-aminomethyl]-benzylalkohol nach Anspruch 1, dadurch gekennzeichnet, dass man als Ausgangsstoffe α-[N-[2-(2,3-Dihydro-2,2-dimethyl-4-oxo-4H-1,3-benzoxazin-6-yloxy)-1-methyl-äthyl]-aminomethyl]-benzylalkohol und Isopropylamin verwendet.

3. Verfahren zur Herstellung von α-[N-[2-(4-Carbamoyl-3-hydroxy-phenoxy)-1-methyl-äthyl]-aminomethyl]-benzylalkohol nach Anspruch 1, dadurch gekennzeichnet, dass man als Ausgangsstoffe α-[N-[2-(2,3-Dihydro-2,2-dimethyl-4-oxo-4H-1,3-benzoxazin-7-yloxy)-1-methyl-äthyl]-aminomethyl]-benzylalkohol und Isopropylamin verwendet.

4. Verfahren zur Herstellung von α-[N-[2-(3-Carbamoyl-4-hydroxy-phenoxy)-äthyl]-aminomethyl]-benzylalkohol nach Anspruch 1, dadurch gekennzeichnet, dass man als ausgangsstoffe α-(Aminomethyl)-benzylalkohol und 2,3-Dihydro-2,2-dimethyl-6-(2-bromäthoxy)-4H-1,3-benzoxazin-4-on verwendet.

5. Verfahren zur Herstellung von α-[N-[2-(4-Carbamoyl-3-hydroxy-phenoxy)-äthyl]-aminomethyl]-benzylalkohol nach Anspruch 1, dadurch gekennzeichnet, dass man als Ausgangsstoffe N-[2-(4-Carbamoyl-3-hydroxy-phenoxy)-äthyl]-N-(2-hydroxy-2-phenyl-äthyl)-benzylamin verwendet.

6. Verfahren zur Herstellung von α-[N-[2-(3-Carbamoyl-4-hydroxy-phenoxy)-1-methyl-äthyl]-aminomethyl]-3-pyridinmethanol nach Anspruch 1, dadurch gekennzeichnet, dass man als Ausgangsstoffe α-(Aminomethyl)-3-pyridinmethanol und 2,3-Dihydro-2,2-dimethyl-6-(2-oxo-propoxy)-4H-1,3-benzoxazin-4-on verwendet.

7. Verfahren zur Herstellung von α-[N-[2-(3-Carbamoyl-4-hydroxy-phenoxy)-1-methyl-äthyl]-aminomethyl]-2-pyridinmethanol nach Anspruch 1, dadurch gekennzeichnet, dass man als Ausgangsstoffe α-(Aminomethyl)-2-pyridinmethanol und 2,3-Dihydro-2,2-dimethyl-6-(2-oxo-propoxy)-4H-1,3-benzoxazin-4-on verwendet.

8. Verfahren zur Herstellung von α-[N-[2-(3-Carbamoyl-4-hydroxy-phenoxy)-1-methyl-äthyl]-aminomethyl]-4-hydroxy-benzylalkohol nach Anspruch 1, dadurch gekennzeichnet, dass man als Ausgangsstoff 4-Benzyloxy-α-[N-[2-(3-Carbamoyl-4-hydroxy-phenoxy)-1-methyl-äthyl]-aminomethyl]-benzylalkohol verwendet.

9. Verfahren zur Herstellung von α-[N-[2-(3-Carbamoyl-4-hydroxy-phenoxy)-1-methyl-äthyl]-aminomethyl]-benzylalkohol nach Anspruch 1, dadurch gekennzeichnet, dass man als Ausgangsstoff die aus 5-(2-Oxo-propoxy)-salicylamid und α-(Aminomethyl)-benzylalkohol gebildete Schiffsche Base verwendet.

10. Verfahren zur Herstellung von R-(-)-α-[N-[2-(3-Carbamoyl-4-hydroxy-phenoxy)-1-methyl-äthyl]-aminomethyl]-benzylalkohol nach Anspruch 1, dadurch gekennzeichnet, dass man als Ausgangsstoff die aus R-(-)-α-(Aminomethyl)-benzylalkohol und 5-(2-Oxo-propoxy)-salicylamid gebildete Schiffsche Base verwendet.

11. Verfahren zur Herstellung von α-[N-[2-(3-Carbamoyl-4-hydroxy-phenoxy)-äthyl]-aminomethyl]-4-hydroxybenzylalkohol nach Anspruch 1, dadurch gekennzeichnet, dass man als Ausgangsstoff. 4-Benzyloxy-α-[N-[2-(3-carbamoyl-4-hydroxy-phenoxy)-äthyl]-aminomethyl]-benzylalkohol verwendet.

12. Verfahren zur Herstellung von α-[N-[2-(2-Carbamoyl-3-hydroxy-phenoxy)-äthyl]-aminomethyl]-benzylalkohol nach Anspruch 1, dadurch gekennzeichnet, dass man als Ausgangsstoff 6-(2-Bromäthoxy)-salicylamid und α-(Aminomethyl)-benzylalkohol verwendet.

13. Verfahren zur Herstellung von α-[N-[3-(4-Carbamoyl-3-hydroxy-phenoxy)-propyl]-aminomethyl]-benzylalkohol nach Anspruch 1, dadurch gekennzeichnet, dass man als Ausgangsstoff N-[3-(4-Carbamoyl-3-hydroxy-phenoxy)-propyl]-N-(2-hydroxy-2-phenyl-äthyl]-benzylamin verwendet.

14. Verfahren zur Herstellung von α-[N-[2-(3-Carbamoyl-4-hydroxy-phenoxy)-1-methyl-äthyl]-aminomethyl]-benzylalkohol nach Anspruch 1, dadurch gekennzeichnet, dass man als Ausgangsstoffe α-[N-[2-(3-Carbomethoxy-4-hydroxy-phenoxy)-1-methyl-äthyl]-aminomethyl]-benzylalkohol und Ammoniak verwendet.

15. Verfahren zur Herstellung von α-[N-[2-(3-Carbamoyl-4-hydroxy-phenoxy)-äthyl]-aminomethyl]-benzylalkohol nach Anspruch 1, dadurch gekennzeichnet, dass man als Ausgangsstoffe Phenylglyoxal und 5-(2-Aminoäthoxy)-salicylamid verwendet.

16. Verfahren zur Herstellung von α-[N-[2-(3-Carbamoyl-4-hydroxy-phenoxy)-äthyl]-aminomethyl]-2-pyridinmethanol nach Anspruch 1, dadurch gekennzeichnet, dass man als Ausgangsstoffe α-(Aminomethyl)-2-pyridinmethanol und 2,3-Dihydro-2,2-dimethyl-6-(2-bromäthoxy)-4H-1,3-benzoxazin-4-on verwendet.

17. Verfahren zur Herstellung von α-[N-[2-(4-Carbamoyl-3-hydroxy-phenoxy)-1-methyl-äthyl]-aminomethyl]-benzylalkohol nach Anspruch 1, dadurch gekennzeichnet, dass man als Ausgangsstoffe Phenyläthylenoxid und 4-(2-Aminopropoxy)-salicylamid verwendet.

18. Verfahren zur Herstellung von α-[N-[2-(3-Carbamoyl-4-hydroxy-phenoxy)-äthyl]-aminomethyl]-2-furanmethanol nach Anspruch 1, dadurch gekennzeichnet, dass man als Ausgangsstoffe α-(Aminomethyl)-2-furanmethanol und 2,3-Dihydro-2,2-dimethyl-6-(2-bromäthoxy)-4H-1,3-benzoxazin-4-on verwendet.

19. Verfahren zur Herstellung von α-[N-[2-(3-Carbamoyl-4-hydroxy-phenoxy)-äthyl]-aminomethyl]-2-thiophen-methanol nach Anspruch 1, dadurch gekennzeichnet, dass man als Ausgangsstoffe α-(Aminomethyl)-2-thiophen-methanol und 2,3-Dihydro-2,2-dimethyl-6-(2-bromäthoxy)-4H-1,3-benzoxazin-4-on verwendet.

20. Verfahren zur Herstellung von S-(+)-α-[N-[2-(3-Carbamoyl-4-hydroxy-phenoxy)-1-methyl-äthyl]-aminomethyl]-benzylalkohol nach Anspruch 1, dadurch gekennzeichnet, dass man als Ausgangsstoffe S-(+)-α-(Aminomethyl)-benzylalkohol und 5-(2-Oxo-propoxy)-salicylamid verwendet.

21. Verfahren zur Herstellung von α-[N-[2-(4-Carbamoyl-3-hydroxy-phenoxy)-äthyl]-aminomethyl]-4-hydroxy-benzylalkohol nach Anspruch 1, dadurch gekennzeichnet, dass man als Ausgangsstoff 4-Benzyloxy-α-[N-[2-(4-Carbamoyl-3-hydroxy-phenoxy)-äthyl-aminomethyl]-benzylalkohol verwendet.

22. Verfahren zur Herstellung von α-[N-[2-(3-Carbamoyl-4-hydroxy-phenoxy)-äthyl]-aminomethyl]-benzylalkohol nach Anspruch 1, dadurch gekennzeichnet, dass man als Ausgangsstoff α-[N-Benzyl-N-[2-(3-carbamoyl-4-hydroxy-phenoxy)-äthyl]-aminomethyl]-benzylalkohol verwendet.

23. Verfahren zur Herstellung von α-[N-[3-(3-Carbamoyl-4-hydroxy-phenoxy)-propyl]-aminomethyl]-benzylalkohol nach Anspruch 1, dadurch gekennzeichnet, dass man als Ausgangsstoffe α-(Aminomethyl)-benzylalkohol und 6-(3-Brompropoxy)-2,3-dihydro-2,2-dimethyl-4H-1,3-benzoxazin-4-on verwendet.

24. Verfahren zur Herstellung von α-[N-[3-(3-Carbamoyl-4-hydroxy-phenyl)-propyl]-aminomethyl]-benzylalkohol nach Anspruch 1, dadurch gekennzeichnet, dass man als Ausgangsstoff α-[N-Benzyl-N-[3-(3-carbamoyl-4-hydroxy-phenyl)-propyl]-aminomethyl]-benzylalkohol verwendet.

25. Verfahren zur Herstellung von α-[N-[2-(3-Carbamoyl-4-hydroxy-phenoxy)-äthyl]-aminomethyl]-3,5-dihydroxy-benzylalkohol nach Anspruch 1, dadurch gekennzeichnet, dass man als Ausgangsstoff N-[2-(3-Carbamoyl-4-hydroxy-phenoxy)-äthyl]-N-[2-(3,5-dibenzyloxyphenyl]-2-hydroxy-äthyl]-benzylamin verwendet.

26. Verfahren zur Herstellung von α-[N-[2-(3-Carbamoyl-4-hydroxy-phenoxy)-äthyl]-aminomethyl]-benzylalkohol nach Anspruch 1, dadurch gekennzeichnet, dass man als Ausgangsstoffe 5-(2-Aminoäthoxy)-salicylamid und α-(Brommethyl)-benzylalkohol verwendet.

27. Verfahren zur Herstellung von α-[N-[2-(3-Carbamoyl-4-hydroxy-phenoxy)-äthyl]-aminomethyl]-benzylalkohol nach Anspruch 1, dadurch gekennzeichnet, dass man als Ausgangsstoffe Phenyläthylenoxid und 5-(2-Aminoäthoxy)-salicylamid verwendet.

28. Verfahren nach einem der Ansprüche 1–27, dadurch gekennzeichnet, dass man eine erhaltene Verbindung der Formel I in ein pharmazeutisch annehmbares, nicht-toxisches Säureadditionssalz überführt.

**Claims for the Contracting states BE, CH, PE, FR, GB, IT, LU, NL, SE**

1. A N-alkylated aminoalcohol of the formula

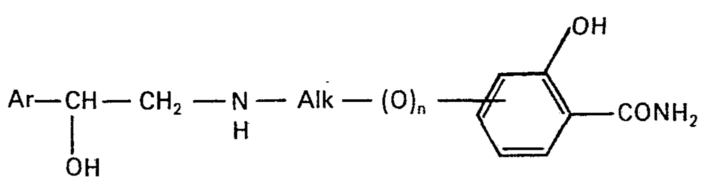

(I)

in which Ar is a radical of aromatic character which is unsubstituted or substituted by hydroxyl, n has the values nought or 1 and Alk is an alkylene radical having 2 to 5 carbon atoms and the nitrogen atom and the oxygen atom or, if n is nought, the phenyl radical are separated from one another by at least 2 carbon atoms, in the form of a mixture of racemates, a stereoisomeric (diastereomeric) racemate or an optical antipode.

2. A compound of the formula I, in which Ar is phenyl, 2- or 3- or 4-pyridyl, 3- or 4- or 6-pyridazinyl, 2- or 4- or 5-pyrimidinyl or 2-pyrazinyl, 2- or 3-furyl, 2-or 3-pyrryl, 2- or 3-thienyl, 2- or 4-oxazolyl, 2- or 4-thiazolyl, 3- or 5-pyrazolyl, 2- or 4-imidazolyl, 1,2,4-thiadiazol-3- or 5-yl or 1,3,4-thiadiazol-2-yl, which radicals are in each case unsubstituted or substituted by 1 or 2 hydroxyl groups, and Alk is an alkylene radical having 2 to 4 carbon atoms and the nitrogen atom and the oxygen atom or, if n is nought, the phenyl radical are separated from one another by 2 to 3 carbon atoms, and n is nought or 1.

3. A compound of the formula I in which Ar is phenyl, 2- or 3-pyridyl, 2- or 3-furyl or 2- or 3-thienyl, which radicals are in each case unsubstituted or substituted by 1 or 2 hydroxyl groups, and Alk is an alkylene radical having 2 or 3 carbon atoms, and the nitrogen atom and the oxygen atom or, if n is nought, the phenyl radical are separated from one another by 2 to 3 carbon atoms, and n is nought or 1.

4. A compound of the formula I in which Ar is phenyl, 2- or 3-pyridyl, 2-furyl or 2-thienyl, which radicals are in each case unsubstituted or substituted by 1 or 2 hydroxyl groups, and Alk is an alkylene radical having 2 or 3 carbon atoms, and the nitrogen atom and the oxygen atom or, if n is nought, the phenyl radical are separated from one another by 2 or 3 carbon atoms, and n is nought or 1.

5. α-[N-[2-(3-Carbamoyl-4-hydroxy-phenoxy)-1-methyl-ethyl]-aminomethyl]-benzyl alcohol as the enantiomer pair with a melting point of 166–168°C.

6. α-[N-[2-(3-Carbamoyl-4-hydroxy-phenoxy)-1-methyl-ethyl]-aminomethyl]-benzyl alcohol as the enantiomer pair with a melting point of 152–154°C.

7. α-[N-[2-(3-Carbamoyl-4-hydroxy-phenoxy)-ethyl]-aminomethyl]-benzyl alcohol.

8. α-[N-[2-(4-Carbamoyl-3-hydroxy-phenoxy)-ethyl]-aminomethyl]-4-hydroxy-benzyl alcohol.

9. A salt of a compound of Claims 1 to 8.

10. A pharmaceutically acceptable, non-toxic acid addition salt of a compound of Claims 1 to 8.

11. A pharmaceutical preparation containing a compound of Claims 1 to 8 and 10, together with inert auxiliaries and carriers.

12. A compound of the formula I as a pharmaceutical active substance.

13. A compound of the formula I as stimulator of cardiac β-receptors.

14. The use of a compound of the formula I for producing pharmaceutical preparations.

15. A compound of the formula I for use in combating cardiac insufficiency.

16. A process for the preparation of a N-alkylated aminoalcohol of the formula

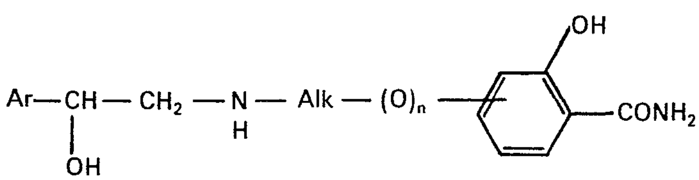

(I)

in which Ar is a radical of aromatic character which is unsubstituted or substituted by hydroxyl, n has the values nought or 1 and Alk is an alkylene radical having 2 to 5 carbon atoms and the nitrogen atom and the oxygen atom or, if n is nought, the phenyl radical are separated from one another by at least 2 carbon atoms, or a salt thereof, which comprises

a) reacting a compound of the formula

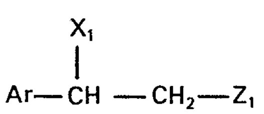

(II)

with a compound of the formula

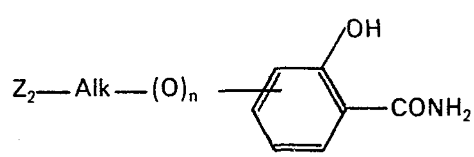

(III)

in which formulae one of the groups $Z_1$ and $Z_2$ is a reactive esterified hydroxyl group and the other is a primary amino group and $X_1$ is hydroxyl, or $X_1$ and $Z_1$ together are an epoxy group and $Z_2$ is a primary amino group, and Ar, Alk and n are as defined above, or

b) in a compound of the formula

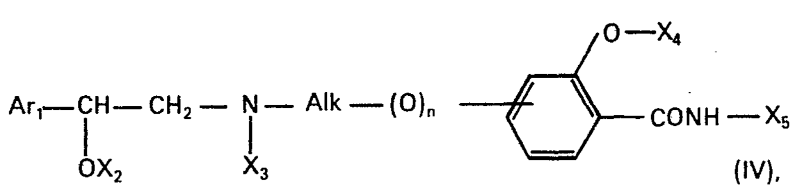

(IV),

in which $Ar_1$ has the meaning defined for Ar or is a radical Ar which is substituted by 1 to 2 groups which can be converted to hydroxyl and $X_2$, $X_3$ and $X_4$ are each hydrogen or a substituent replaceable by hydrogen and $X_5$ is hydrogen, or $X_2$ and $X_3$ and/or $X_4$ and $X_5$ together are a divalent radical replaceable by two hydrogen atoms, with the proviso that at least one of the radicals $X_2$, $X_3$

and $X_4$ differs from hydrogen, or at least $Ar_1$ is a radical Ar which is substituted by 1 to 2 groups which can be converted to hydroxyl, or at least $X_2$ and $X_3$ together or $X_4$ and $X_5$ together are a divalent radical replaceable by two hydrogen atoms, or in a salt thereof, replacing by hydrogen the $X_2$, $X_3$, $X_4$ or $X_4$ and $X_5$ together which differ from hydrogen and/or converting substituted hydroxyl present in a radical $Ar_1$ to free hydroxyl, or
c) in a compound of the formula

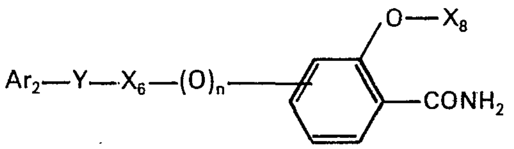
(V),

in which $X_6$ is a reducible group of the formulae $-CH=N-Alk$ (Va) or $-CH_2-N=Alk_1-$ (Vb) or $-C(=X_7)-N(X_8)-Alk$ (Vc) or $-CH_2-N(X_8)-C(=X_7)-Alk_2$ (Vd) or a group $-CH_2-N(X_8)-Alk$ (Ve), in which formulae $Alk_1$ is an alkylylidene radical corresponding to a radical Alk and $Alk_2$ corresponds to a radical Alk shortened by a methylene group bonded to the nitrogen atom, $X_7$ is an oxo or thioxo radical and $X_8$ is hydrogen or a radical replaceable by hydrogen under the conditions for the reduction of $X_6$ and/or Y, and Y is a radical of the formula $-CO-$(Vf) or $-CH(OX_8)-$ (Vg), in which $X_8$ is as defined above, $Ar_2$ corresponds to a radical Ar but can carry one or two groups $OX_8$, in which $X_8$ is as defined above, in place of one or two hydroxyls, and n is nought or 1 and, in every case, $X_6$ is a reducible group Va to Vd and/or Y is a carbonyl group Vf, reducting this group or groups and, in the same operation, replacing by hydrogen the groups $X_8$ which differ from hydrogen, or
d) reacting a compound of the formula

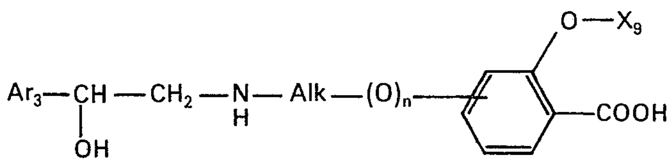
(VI)

in which $Ar_3$ has the meaning defined for Ar or is a radical Ar which is substituted by 1 to 2 groups convertible to hydroxyl by ammonolysis and $X_9$ is hydrogen or a group detachable by ammonolysis, or a reactive derivative of one of the carboxylic acids defined by formula VI, with ammonia and, at the same time, detaching any radicals $X_9$ which may be present and replacing these by hydrogen, and/or, if desired, converting the resulting free compound into a salt or converting a resulting salt into the free compound and/or, if desired, separating the resulting mixture of racemates into the two stereoisomeric (diastereomeric) racemates or resolving a resulting racemate into the optical antipodes.

17. A process according to claim 16, wherein a resulting compound of the formula I is converted to a pharmaceutically acceptable, non-toxic acid addition salt.

**Claims for the Contracting state AT**

1. A process for the preparation of a N-alkylated aminoalcohol of the formula

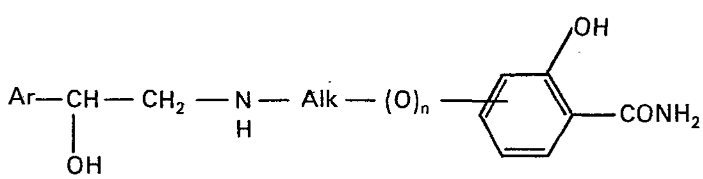
(I)

in which Ar is a radical of aromatic character which is unsubstituted or substituted by hydroxyl, n has the values nought or 1 and Alk is an alkylene radical having 2 to 5 carbon atoms and the nitrogen atom and the oxygen atom or, if n is nought, the phenyl radical are separated from one another by at least 2 carbon atoms, or a salt thereof, which comprises
a) reacting a compound of the formula

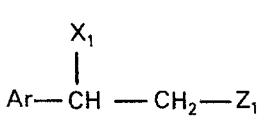
(II)

with a compound of the formula

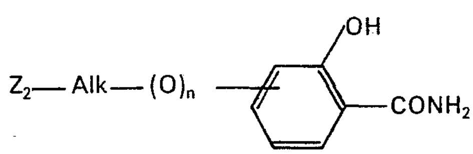
(III)

in which formulae one of the groups $Z_1$ and $Z_2$ is a reactive esterified hydroxyl group and the other is a primary amino group and $X_1$ is hydroxyl, or $X_1$ and $Z_1$ together are an epoxy group and $Z_2$ is a primary amino group, and Ar, Alk and n are as defined above, or
b) in a compound of the formula

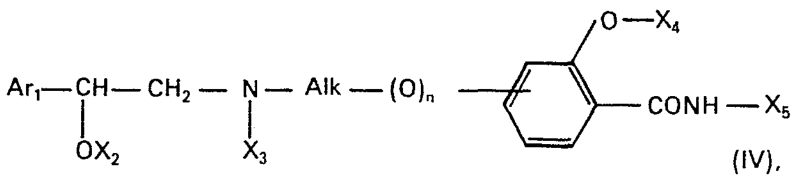

(IV),

in which $Ar_1$ has the meaning defined for Ar or is a radical Ar which is substituted by 1 to 2 groups which can be converted to hydroxyl and $X_2$, $X_3$ and $X_4$ are each hydrogen or a substituent replaceable by hydrogen and $X_5$ is hydrogen, or $X_2$ and $X_3$ and/or $X_4$ and $X_5$ together are a divalent radical replaceable by two hydrogen atoms, with the proviso that at least one of the radicals $X_2$, $X_3$ and $X_4$ differs from hydrogen, or at least $Ar_1$ is a radical Ar which is substituted by 1 to 2 groups which can be converted to hydroxyl, or at least $X_2$ and $X_3$ together or $X_4$ and $X_5$ together are a divalent radical replaceable by two hydrogen atoms, or in a salt thereof, replacing by hydrogen the $X_2$, $X_3$, $X_4$ or $X_4$ and $X_5$ together which differ from hydrogen and/or converting substituted hydroxyl present in a radical $Ar_1$ to free hydroxyl, or

c) in a compound of the formula

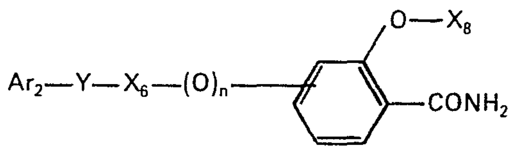

(V),

in which $X_6$ is a reducible group of the formulae $-CH{=}N-Alk$ (Va) or $-CH_2-N{=}Alk_1-$ (Vb) or $-C(=X_7)-N(X_8)-Alk$ (Vc) or $-CH_2-N(X_8)-C(=X_7)-Alk_2$ (Vd) or a group $-CH_2-N(X_8)-Alk$ (Ve), in which formulae $Alk_1$ is an alkylylidene radical corresponding to a radical Alk and $Alk_2$ corresponds to a radical Alk shortened by a methylene group bonded to the nitrogen atom, $X_7$ is an oxo or thioxo radical and $X_8$ is hydrogen or a radical replaceable by hydrogen under the conditions for the reduction of $X_6$ and/or Y, and Y is a radical of the formula -CO- (Vf) or $-CH(OX_8)-$ (Vg), in which $X_8$ is as defined above, $Ar_2$ corresponds to a radical Ar but can carry one or two groups $OX_8$, in which $X_8$ is as defined above, in place of one or two hydroxyls, and n is nought or 1 and, in every case, $X_6$ is a reducible group Va to Vd and/or Y is a carbonyl group Vf, reducing this group or groups and, in the same operation, replacing by hydrogen the groups $X_8$ which differ from hydrogen, or

d) reacting a compound of the formula

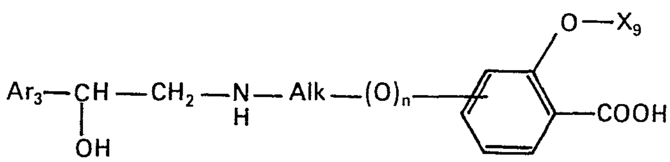

(VI)

in which $Ar_3$ has the meaning defined for Ar or is a radical Ar which is substituted by 1 to 2 groups convertible to hydroxyl by ammonolysis and $X_9$ is hydrogen or a group detachable by ammonolysis, or a reactive derivative of one of the carboxylic acids defined by formula VI, with ammonia and, at the same time, detaching any radicals $X_9$ which may be present and replacing these by hydrogen, and/or, if desired, converting the resulting free compound into a salt or converting a resulting salt into the free compound and/or, if desired, separating the resulting mixture of racemates into the two stereoisomeric (diastereomeric) racemates or resolving a resulting racemate into the optical antipodes.

2. A process according to Claim 1 for producing α-[N-[2-(3-carbamoyl-4-hydroxy-phenoxy)-1-methyl-ethyl]-aminomethyl]-benzyl alcohol, wherein the starting materials used are α-[N-[2-(2,3-dihydro-2,2-dimethyl-4-oxo-4H-1,3-benzoxazin-6-yloxy)-1-methyl-ethyl]-aminomethyl]-benzyl alcohol and isopropylamine.

3. A process according to Claim 1 for producing α-[N-[2-(4-carbamoyl-3-hydroxyphenoxy)-1-methyl-ethyl]-aminomethyl]-benzyl alcohol, wherein the starting materials used are α-[N-[2-(2,3-dihydro-2,2-dimethyl-4-oxo-4H-1,3-benzoxazin-7-yloxy)-1-methyl-ethyl]-aminomethyl]-benzyl alcohol and isopropylamine.

4. A process according to Claim 1 for producing α-[N-[2-(3-carbamoyl-4-hydroxy-phenoxy)-ethyl]-aminomethyl]-benzyl alcohol, wherein the starting materials used are α-(aminomethyl)-benzyl alcohol and 2,3-dihydro-2,2-dimethyl-6-(2-bromoethoxy)-4H-1,3-benzoxazin-4-one.

5. A process according to Claim 1 for producing α-[N-[2-(4-carbamoyl-3-hydroxy-phenoxy)-ethyl]-aminomethyl]-benzyl alcohol, wherein the starting materials used are N-[2-(4-carbamoyl-3-hydroxy-phenoxy)-ethyl]-N-(2-hydroxy-2-phenyl-ethyl)-benzylamine.

6. A process according to Claim 1 for producing α-[N-[2-(3-carbamoyl-4-hydroxy-phenoxy)-1-methyl-ethyl]-aminomethyl]-3-pyridinemethanol, wherein the starting materials used are α-(aminomethyl)-3-pyridinemethanol and 2,3-dihydro-2,2-dimethyl-6-(2-oxo-propoxy)-4H-1,3-benzoxazin-4-one.

7. A process according to Claim 1 for producing α-[N-[2-(3-carbamoyl-4-hydroxy-phenoxy)-1-methyl-ethyl]-aminomethyl]-2-pyridinemethanol, wherein the starting materials used are α-(aminomethyl)-2-pyridinemethanol and 2,3-dihydro-2,2-dimethyl-6-(2-oxo-propoxy)-4H-1,3-benzoxazin-4-one.

8. A process according to Claim 1 for producing α-[N-[2-(3-carbamoyl-4-hydroxy-phenoxy)-1-methyl-ethyl]-aminomethyl]-4-hydroxybenzyl alcohol, wherein a starting material used is 4-benzyloxy-α-[N-[2-(3-carbamoyl-4-hydroxyphenoxy)-1-methyl-ethyl]-aminomethyl]-benzyl alcohol.

9. A process according to Claim 1 for producing α-[N-[2-(3-carbamoyl-4-hydroxy-phenoxy)-1-methyl-ethyl]-aminomethyl]-benzyl alcohol, wherein a starting material used is the Schiff base formed from 5-(2-oxo-propoxy)-salicylamide and α-(aminomethyl)-benzyl alcohol.

10. A process according to Claim 1 for producing R-(-)-α-[N-[2-(3-carbamoyl-4-hydroxyphenoxy)-1-methyl-ethyl]-aminomethyl]-benzyl alcohol, wherein a starting material used is the Schiff base formed from R-(-)-α-(aminomethyl)-benzyl alcohol and 5-(2-oxo-propoxy)-salicylamide.

11. A process according to Claim 1 for producing α-[N-[2-(3-carbamoyl-4-hydroxy-phenoxy)-ethyl]-aminomethyl]-4-hydroxybenzyl alcohol, wherein a starting material used is 4-benzyloxy-α-[N-[2-(3-carbamoyl-4-hydroxy-phenoxy)-ethyl]-aminomethyl]-benzyl alcohol.

12. A process according to Claim 1 for producing α-[N-[2-(2-carbamoyl-3-hydroxy-phenoxy)-ethyl]-aminomethyl]-benzyl alcohol, wherein a starting material used is 6-(2-bromoethoxy)-salicylamide and α-(aminomethyl)-benzyl alcohol.

13. A process according to Claim 1 for producing α-[N-[3-(4-carbamoyl-3-hydroxy-phenoxy)-propyl]-aminomethyl]-benzyl alcohol, wherein a starting material used is N-[3-(4-carbamoyl-3-hydroxy-phenoxy)-propyl]-N-(2-hydroxy-2-phenyl-ethyl)-benzylamine.

14. A process according to Claim 1 for producing α-[N-[2-(3-carbamoyl-4-hydroxy-phenoxy)-1-methyl-ethyl]-aminomethyl]-benzyl alcohol, wherein the starting materials used are α-[N-[2-(3-carbomethoxy-4-hydroxy-phenoxy)-1-methyl-ethyl]-aminomethyl]-benzyl alcohol and ammonia.

15. A process according to Claim 1 for producing α-[N-[2-(3-carbamoyl-4-hydroxy-phenoxy)-ethyl]-aminomethyl]-benzyl alcohol, wherein the starting materials used are phenylglyoxal and 5-(2-aminoethoxy)-salicylamide.

16. A process according to Claim 1 for producing α-[N-[2-(3-carbamoyl-4-hydroxy-phenoxy)-ethyl]-aminomethyl]-2-pyridinemethanol, wherein the starting materials used are α-(aminomethyl)-2-pyridinemethanol and 2,3-dihydro-2,2-dimethyl-6-(2-bromoethoxy)-4H-1,3-benzoxazin-4-one.

17. A process according to Claim 1 for producing α-[N-[2-(4-carbamoyl-3-hydroxy-phenoxy)-1-methyl-ethyl]-aminomethyl]-benzyl alcohol, wherein the starting materials used are phenylethylene oxide and 4-(2-amino-propoxy)-salicylamide.

18. A process according to Claim 1 for producing α-[N-[2-(3-carbamoyl-4-hydroxy-phenoxy)-ethyl]-aminomethyl]-2-furanmethanol, wherein the starting materials used are α-(aminomethyl)-2-furanmethanol and 2,3-dihydro-2,2-dimethyl-6-(2-bromoethoxy)-4H-1,3-benzoxazin-4-one.

19. A process according to Claim 1 for producing α-[N-[2-(3-carbamoyl-4-hydroxy-phenoxy)-ethyl]-aminomethyl]-2-thiophenemethanol, wherein the starting materials used are α-(aminomethyl)-2-thiophenemethanol and 2,3-dihydro-2,2-dimethyl-6-(2-bromoethoxy)-4H-1,3-benzoxazin-4-one.

20. A process according to Claim 1 for producing S-(+)-α-[N-[2-(3-carbamoyl-4-hydroxyphenoxy)-1-methyl-ethyl]-aminomethyl]-benzyl alcohol, wherein the starting materials used are S-(+)-α-(aminomethyl)-benzyl alcohol and 5-(2-oxo-propoxy)-salicylamide.

21. A process according to Claim 1 for producing α-[N-[2-(4-carbamoyl-3-hydroxy-phenoxy)-ethyl]-aminomethyl]-4-hydroxy-benzyl alcohol, wherein a starting material used is 4-benzyloxy-α-[N-[2-(4-carbamoyl-3-hydroxy-phenoxy)-ethyl]-aminomethyl]-benzyl alcohol.

22. A process according to Claim 1 for producing α-[N-[2-(3-carbamoyl-4-hydroxy-phenoxy)-ethyl]-aminomethyl]-benzyl alcohol, wherein a starting material used is α-[N-benzyl-N-[2-(3-carbamoyl-4-hydroxy-phenoxy)-ethyl]-aminomethyl]-benzyl alcohol.

23. A process according to Claim 1 for producing α-[N-[3-(3-carbamoyl-4-hydroxy-phenoxy)-propyl]-aminomethyl]-benzyl alcohol, wherein the starting materials used are α-(aminomethyl)-benzyl alcohol and 6-(3-bromopropoxy)-2,3-dihydro-2,2-dimethyl-4H-1,3-benzoxazin-4-one.

24. A process according to Claim 1 for producing α-[N-[3-(3-carbamoyl-4-hydroxy-phenyl)-propyl]-aminomethyl]-benzyl alcohol, wherein a starting material used is α-[N-benzyl-N-[3-(3-carbamoyl-4-hydroxy-phenyl)-propyl]-aminomethyl]-benzyl alcohol.

25. A process according to Claim 1 for producing α-[N-[2-(3-carbamoyl-4-hydroxy-phenoxy)-ethyl]-aminomethyl]-3,5-dihydroxy-benzyl alcohol, wherein a starting material used is N-[2-(3-carbamoyl-4-hydroxy-phenoxy)-ethyl]-N-[2-(3,5-dibenzyloxyphenyl)-2-hydroxy-ethyl]-benzylamine.

26. A process according to Claim 1 for producing α-[N-[2-(3-carbamoyl-4-hydroxy-phenoxy)-ethyl]-aminomethyl]-benzyl alcohol, wherein the starting materials used are 5-(2-amino-ethoxy)-salicylamide and α-(bromomethyl)-benzyl alcohol.

27. A process according to Claim 1 for producing α-[N-[2-(3-carbamoyl-4-hydroxy-phenoxy)-ethyl]-aminomethyl]-benzyl alcohol, wherein the starting materials used are phenylethylene oxide

and 5-(2-aminoethoxy)-salicylamide.

28. A process according to any one of Claims 1-27, wherein a resulting compound of the formula I is converted into a pharmaceutically acceptable, non-toxic acid addition salt.

**Revendications pour les Etats contractants BE, CH, DE, FR, GB, IT, LU, NL, SE**

1. Aminoalcools N-alcoylés de formule

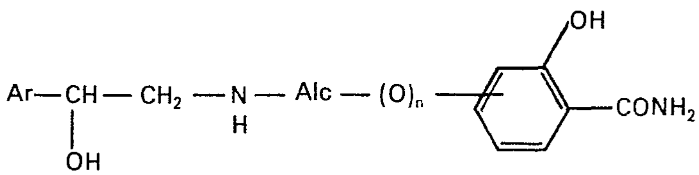

(I)

où Ar représente un radical non substitué ou substitué par hydroxy de caractère aromatique, n les nombres zéro ou 1 et Alc un radical alcoylène ayant de 2 à 5 atomes de carbone, où l'atome d'azote et l'atome d'oxygène ou, si n vaut zéro, le radical phényle sont séparés l'un de l'autre par au moins deux atomes de carbone sous forme de mélanges de racémates, de racémates stéréoisomères (diastéréoisomères) ou d'antipodes optiques.

2. Composés de formule I, où Ar représente un phényle non substitué ou substitué par un ou deux groupes hydroxy, un 2-, 3- ou 4-pyridyle, un 3-, 4- ou 6-pyridazinyle, un 2-, 4- ou 5-pyrimidinyle ou un 2-pyrazinyle, un 2-, ou 3-furyle, un 2- ou 3-pyrryle, un 2- ou 3-thiényle, un 2- ou 4-oxazolyle, un 2- ou 4-thiazolyle, un 3- ou 5-pyrazolyle, un 2- ou 4-imidazolyle, un 1,2,4-thiadiazol-3- ou 5-yle ou un 1,3,4-thiadiazol-2-yle et Alc un radical alcoylène ayant de 2 à 4 atomes de carbone, l'atome d'azote et l'atome d'oxygène ou, si n vaut zéro, le radical phényle, étant séparés l'un de l'autre par 2 à 3 atomes de carbone, et n vaut zéro ou 1.

3. Composés de formule I, où Ar représente un phényle non substitué ou substitué par un ou deux groupes hydroxy, un 2- ou 3-pyridyle, un 2- ou 3-furyle ou un 2- ou 3-thiényle et Alc un radical alcoylène ayant de 2 à 3 atomes de carbone, où l'atome d'azote et l'atome d'oxygène ou, si n vaut zéro, le radical phényle, sont séparés l'un de l'autre par 2 à 3 atomes de carbone, et n vaut zéro ou 1.

4. Composés de formule I, où Ar représente un phényle non substitué ou substitué par un ou deux groupes hydroxy, un 2- ou 3-pyridyle, un 2-furyle ou un 2-thiényle et Alc un radical alcoylène ayant de 2 à 3 atomes de carbone, l'atome d'azote et l'atome oxygène ou, si n vaut zéro, le noyau phényle, étant séparés l'un de l'autre par 2 à 3 atomes de carbone, et n vaut zéro ou 1.

5. α-[N-[2-(3-carbamoyl-4-hydroxy-phénoxy)-1-méthyl-éthyl]-aminométhyl]-benzylalcool sous forme de paire d'énantiomères de $P_f$ 166-168°.

6. α-[N-[2-(3-carbamoyl-4-hydroxy-phénoxy)-1-méthyl-éthyl]-aminométhyl]-benzylalcool sous forme de paire d'énantiomères de $P_f$ 152-154°.

7. α-[N-[2-(3-carbamoyl-4-hydroxy-phénoxy)-éthyl]-aminométhyl]-benzylalcool.

8. α-[N-[2-(4-carbamoyl-3-hydroxy-phénoxy)-éthyl]-aminométhyl]-4-hydroxy-benzylalcool.

9. Les sels des composés des revendications 1 à 8.

10. Sels d'addition acides non toxiques pharmaceutiquement acceptables des composés des revendications 1 à 8.

11. Préparations pharmaceutiques contenant un composé des revendications 1 à 8 et 10 avec des additifs et supports inertes.

12. Composés de formule I comme médicaments.

13. Composés de formule I comme stimulateurs des β-récepteurs cardiaques.

14. Application des composés de formule I à la préparation de médicaments.

15. Composés de formule I aux fins d'application dans la lutte contre l'insuffisance cardiaque.

16. Procédé de préparation d'aminoalcools N-alcoylés de formule

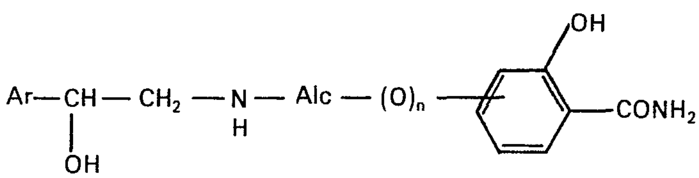

(I)

où Ar représente un radical non substitué ou substitué par hydroxy de caractère aromatique, n les nombres zéro ou 1 et Alc un radical alcoylène ayant de 2 à 5 atomes de carbone, où l'atome d'azote et l'atome d'oxygène ou, si n vaut zéro, le radical phényle sont séparés l'un de l'autre par au moins deux atomes de carbone et de leurs sels, caractérisé en ce qu'on procède

a) en faisant réagir un composé de formule

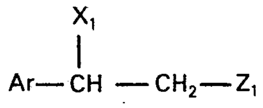

(II)

avec un composé de formule

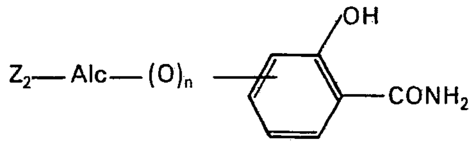

(III)

où l'un des groupes $Z_1$ et $Z_2$ représente un groupe hydroxy estérifié réactif et l'autre le groupe amino primaire, et $X_1$ représente un hydroxy, ou bien où $X_1$ et $Z_1$ représentent ensemble le groupe époxy et $Z_2$ représente le groupe amino primaire, Ar, Alc et n ont la signification donnée ci-dessus, ou

b) dans un composé de formule

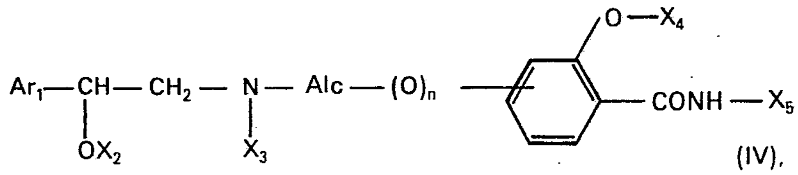

(IV),

où $Ar_1$ a la signification d'Ar ou représente un radical Ar substitué par 1 à 2 groupes transformables en hydroxy, $X_2$, $X_3$ et $X_4$ représentent chacun un hydrogène ou un substituant remplaçable par un hydrogène, et $X_5$ représente un hydrogène, ou $X_2$ et $X_3$ et/ou $X_4$ et $X_5$ représentent ensemble un radical bivalent remplaçable par 2 atomes d'hydrogène, avec la précision qu'au moins l'un des radicaux $X_2$, $X_3$ et $X_4$ est différent d'un hydrogène, ou bien où au moins $Ar_1$ représente un radical Ar substitué par 1 à 2 groupes transformables en hydroxy, ou bien où au moins $X_2$ et $X_3$ ensemble ou $X_4$ et $X_5$ ensemble représentent un radical bivalent remplaçable par 2 atomes d'hydrogène, ou dans un de ses sels, au remplacement du $X_2$, $X_3$ ou $X_4$ qui est différent d'un hydrogène, ou bien de $X_4$ et $X_5$ ensemble par un hydrogène, et/ou en transformant dans un radical $Ar_1$ l'hydroxy substitué présent en un hydroxy libre, ou

c) en réduisant dans un composé de formule

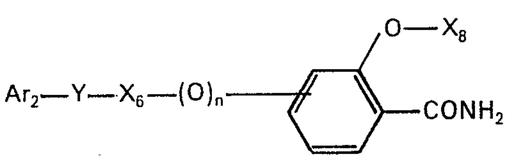

(V)

où $X_6$ est un groupe réductible de formules $-CH = N - Alc-$ (Va), ou $- CH_2-N = Alc_1$ (Vb) ou $-C(=X_7)-N(X_8)-Alc-$ (Vc) ou $-CH_2-N(X_8)-C(=X_7)-Alc_2-$ (Vd) ou un groupe $-CH_2-N(X_8)-Alc-$ (Ve), où $Alc_1$ représente le radical alcoyl-ylidène correspondant à un radical Alc et où $Alc_2$ correspond à un radical Alc raccourci d'un groupe méthylène lié sur l'atome d'azote, $X_7$ représente le radical oxo ou thioxo et $X_8$ un hydrogène ou un radical remplaçable par un hydrogène dans les conditions pour la réduction de $X_6$ et/ou Y et Y représente un radical de formule -CO- (Vf) ou $-CH(OX_8)-$ (Vg) où $X_8$ a la signification donnée ci-dessus, $Ar_2$ correspond à un radical Ar, mais porte cependant éventuellement à la place d'un ou de deux hydroxy un ou deux groupes $OX_8$, où $X_8$ a la signification donnée ci-dessus, et n vaut zéro ou 1, où $X_6$ est toujours un groupe Va à Vd réductible et/ou Y un groupe carbonyle Vf, en réduisant donc ce ou ces groupe(s) et dans le même mode opératoire en remplaçant les groupes $X_8$ différents d'un hydrogène par un hydrogène,

ou

d) en faisant réagir avec de l'ammoniac un composé de formule

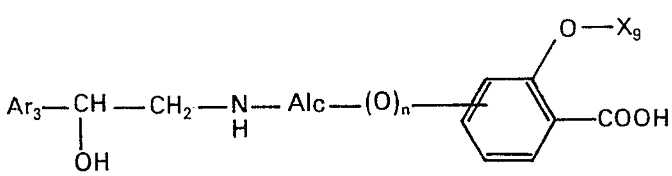

(VI)

où $Ar_3$ a la signification de Ar, ou représente un radical Ar, qui est substitué par un à deux groupes transformables en hydroxy au moyen d'une ammonolyse, $X_9$ représente un hydrogène ou un groupe séparable par ammonolyse, ou un dérivé réactif de l'un des acides carboxyliques définis dans la formule VI, et simultanément en séparant les radicaux $X_9$ éventuellement présents et en les remplaçant par de l'hydrogène, et/ou, si on le désire, en dédoublant un mélange de racémates obtenu pour donner les deux racémates stéréoisomères (diastéréoisomères) ou un racémate obtenu pour donner les antipodes optiques.

17. Procédé selon la revendication 16 caractérisé en ce qu'on transforme un composé de formule I obtenu en un sel d'addition acide non toxique pharmaceutiquement acceptable.

**Revendications pour l'Etat contractant AT**

1. Procédé de préparation d'aminoalcools N-alcoylés de formule

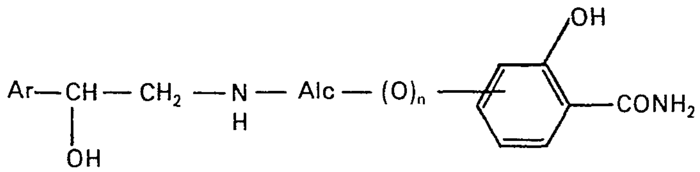 (I)

où Ar représente un radical non substitué ou substitué par hydroxy de caractère aromatique, n les nombres zéro ou 1 et Alc un radical alcoylène ayant de 2 à 5 atomes de carbone, où l'atome d'azote et l'atome d'oxygène ou, si n vaut zéro, le radical phényle sont séparés l'un de l'autre par au moins deux atomes de carbone et de leurs sels, caractérisé en ce qu'on procède

a) en faisant réagir un composé de formule

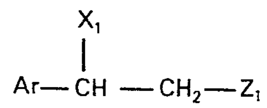 (II)

avec un composé de formule

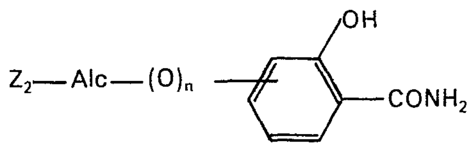 (III)

où l'un des groupes $Z_1$ et $Z_2$ représente un groupe hydroxy estérifié réactif et l'autre le groupe amino primaire, et $X_1$ représente un hydroxy, ou bien où $X_1$ et $Z_1$ représentent ensemble le groupe époxy et $Z_2$ représente le groupe amino primaire, Ar, Alc et n ont la signification donnée ci-dessus, ou

b) dans un composé de formule

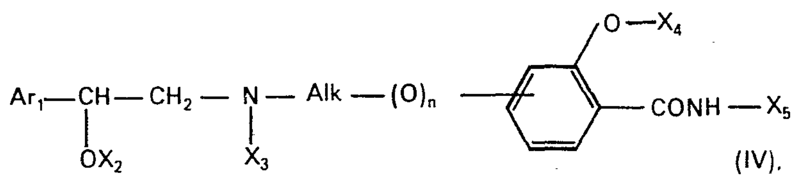 (IV),

où $Ar_1$ a la signification d'Ar ou représente un radical Ar substitué par 1 à 2 groupes transformables en hydroxy, $X_2$, $X_3$ et $X_4$ représentent chacun un hydrogène ou un substituant remplaçable par un hydrogène, et $X_5$ représente un hydrogène, ou $X_2$ et $X_3$ et/ou $X_4$ et $X_5$ représentent ensemble un radical bivalent remplaçable par 2 atomes d'hydrogène, avec la précision qu'au moins l'un des radicaux $X_2$, $X_3$ et $X_4$ est différent d'un hydrogène, ou bien où au moins $Ar_1$ représente un radical Ar substitué par 1 à 2 groupes transformables en hydroxy, ou bien où au moins $X_2$ et $X_3$ ensemble ou $X_4$ et $X_5$ ensemble représentent un radical bivalent remplaçable par 2 atomes d'hydrogène, ou dans un de ses sels, au remplacement du $X_2$, $X_3$, ou $X_4$ qui est différent d'un hydrogène, ou bien de $X_4$ et $X_5$ ensemble par un hydrogène, et/ou en transformant dans un radical $Ar_1$ l'hydroxy substitué présent en un hydroxy libre,

ou

c) en réduisant dans un composé de formule

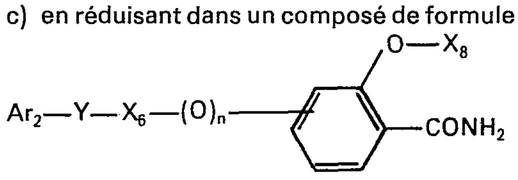 (V)

où $X_6$ est un groupe réductible de formules $-CH=N-Alc-$ (Va), ou $-CH_2-N=Alc_1$ (Vb) ou $-C(=X_7)-N(X_8)-Alc-$ (Vc) ou $-CH_2-N(X_8)-C(=X_7)-Alc_2-$ (Vd) ou un groupe $-CH_2-N(X_8)-Alc-$ (Ve), où $Alc_1$ représente le radical alcoyl-ylidène correspondant à un radical Alc et où $Alc_2$ correspond à un radical Alc raccourci d'un groupe méthylène lié sur l'atome d'azote, $X_7$ représente le radical oxo ou thioxo et $X_8$ un hydrogène ou un radical remplaçable par un hydrogène dans les conditions pour la réduction de $X_6$ et/ou Y et Y représente un radical de formule -CO- (Vf) ou $-CH(OX_8)-$ (Vg) où $X_8$ a la signification donnée ci-dessus, $Ar_2$ correspond à un radical Ar, mais porte cependant éventuellement à la place d'un ou de deux hydroxy un ou deux groupes $OX_8$, où $X_8$ a la signification donnée ci-dessus, et n vaut zéro ou 1, où $X_6$ est toujours un groupe Va à Vd réductible et/ou Y un groupe carbonyle Vf, en réduisant donc ce ou ces groupe(s) et dans le même mode opératoire en remplaçant les groupes $X_8$ différents d'un hydrogène par un hydrogène,

ou

d) en faisant réagir avec de l'ammoniac un composé de formule

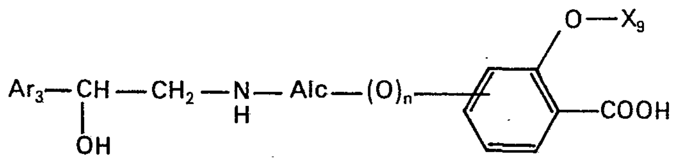

(VI)

où $Ar_3$ a la signification de Ar, ou représente un radical Ar, qui est substitué par un à deux groupes transformables en hydroxy au moyen d'une ammonolyse, $X_9$ représente un hydrogène ou un groupe séparable par ammonolyse, ou un dérivé réactif de l'un des acides carboxyliques définis dans la formule VI, et simultanément en séparant les radicaux $X_9$ éventuellement présents et en les remplaçant par de l'hydrogène, et/ou, si on le désire, en dédoublant un mélange de racémates obtenu pour donner les deux racémates stéréoisomères (diastéréoisomères) ou un racémate obtenu pour donner les antipodes optiques.

2. Procédé de préparation d'α-[N-[2-(3-carbamoyl-4-hydroxy-phénoxy)-1-méthyl-éthyl]-aminométhyl]-benzylalcool selon la revendication 1, caractérisé en ce qu'on utilise comme produits de départ l'α-[N-[2-(2,3-dihydro-2,2-diméthyl-4-oxo-4H-1,3-benzoxazin-6-yloxy)-1-méthyl-éthyl]-aminométhyl]-benzylalcool et l'isopropylamine.

3. Procédé de préparation d'α-[N-[2-(4-carbamoyl-3-hydroxy-phénoxy)-1-méthyl-éthyl]-aminométhyl]-benzylalcool selon la revendication 1, caractérisé en ce qu'on utilise comme produits de départ l'α-[N-[2-(2,3-dihydro-2,2-diméthyl-4-oxo-4H-1,3-benzoxazin-7-yloxy)-1-méthyl-éthyl]-aminométhyl]-benzylalcool et l'isopropylamine.

4. Procédé de préparation d'α-[N-[2-(3-carbamoyl-4-hydroxy-phénoxy)-éthyl]-aminométhyl]-benzylalcool selon la revendication 1, caractérisé en ce qu'on utilise comme produits de départ l'α-(aminométhyl)-benzylalcool et la 2,3-dihydro-2,2-diméthyl-6-(2-brométhoxy)-4H-1,3-benzoxazin-4-one.

5. Procédé de préparation d'α-[N-[2-(4-carbamoyl-3-hydroxy-phénoxy)-éthyl]-aminométhyl]-benzylalcool selon la revendication 1, caractérisé en ce qu'on utilise comme produit de départ la N-[2-(4-carbamoyl-3-hydroxy-phénoxy)-éthyl]-N-(2-hydroxy-2-phényl-éthyl)-benzylamine.

6. Procédé de préparation d'α-[N-[2-(3-carbamoyl-4-hydroxy-phénoxy)-1-méthyl-éthyl]-aminométhyl]-3-pyridine méthanol selon la revendication 1, caractérisé en ce qu'on utilise comme produits de départ l'α-(aminométhyl)-3-pyridineméthanol et la 2,3-dihydro-2,2-diméthyl-6-(2-oxo-propoxy)-4H-1,3-benzoxazin-4-one.

7. Procédé de préparation d'α-[N-[2-(3-carbamoyl-4-hydroxy-phénoxy)-1-méthyl-éthyl]-aminométhyl]-2-pyridineméthanol selon la revendication 1, caractérisé en ce qu'on utilise comme produits de départ l'α-(aminométhyl)-2-pyridineméthanol et la 2,3-dihydro-2,2-diméthyl-6-(2-oxo-propoxy)-4H-1,3-benzoxazin-4-one.

8. Procédé de préparation d'α-[N-[2-(3-carbamoyl-4-hydroxy-phénoxy)-1-méthyl-éthyl]-aminométhyl]-4-hydroxy-benzylalcool selon la revendication 1, caractérisé en ce qu'on utilise comme produit de départ le 4-benzyloxy-α-[N-[2-(3-carbamoyl-4-hydroxy-phénoxy)-1-méthyl-éthyl]-aminométhyl]-benzylalcool.

9. Procédé de préparation d'α-[N-[2-(3-carbamoyl-4-hydroxy-phénoxy)-1-méthyl-éthyl]-aminométhyl]-benzylalcool selon la revendication 1, caractérisé en ce qu'on utilise comme produit de départ la base de Schiff formée à partir du 5-(2-oxo-propoxy)-salicylamide et de l'α-(aminométhyl)-benzylalcool.

10. Procédé de préparation du R-(-)-α[N-[2-(3-carbamoyl-4-hydroxy-phénoxy)-1-méthyl-éthyl]-aminométhyl]-benzylalcool selon la revendication 1, caractérisé en ce qu'on utilise comme produit de départ la base de Schiff formée à partir du R-(-)-α-(aminométhyl)-benzylalcool et de 5-(2-oxo-propoxy)-salicylamide.

11. Procédé de préparation d'α-[N-[2-(3-carbamoyl-4-hydroxy-phénoxy)-éthyl]-aminométhyl]-4-hydroxybenzylalcool selon la revendication 1, caractérisé en ce qu'on utilise comme produit de départ le 4-benzyloxy-α-[N-[2-(3-carbamoyl-4-hydroxy-phénoxy)-éthyl]-aminométhyl]-benzylalcool.

12. Procédé de préparation du α-[N-[2-(2-carbamoyl-3-hydroxy-phénoxy)-éthyl]-aminométhyl]-benzylalcool selon la revendication 1, caractérisé en ce qu'on utilise comme produit de départ le 6-(2-brométhoxy)-salicylamide et l'α-(aminométhyl)-benzylalcool.

13. Procédé de préparation du α-[N-[3-(4-carbamoyl-3-hydroxy-phénoxy)-propyl]-aminométhyl]-benzylalcool selon la revendication 1, caractérisé en ce qu'on utilise comme produit de départ la N-[3-(4-carbamoyl-3-hydroxy-phénoxy)-propyl]-N-(2-hydroxy-2-phényl-éthyl)-benzylamine.

14. Procédé de préparation du α-[N-[2-(3-carbamoyl-4-hydroxy-phénoxy)-1-méthyl-éthyl]-aminométhyl]-benzylalcool selon la revendication 1, caractérisé en ce qu'on utilise comme produit de départ le α-[N-[2-(3-carbométhoxy-4-hydroxy-phénoxy)-1-méthyléthyl]-aminométhyl]-benzylalcool et l'ammoniac.

15. Procédé de préparation du α-[N-[2-(3-carbamoyl-4-hydroxy-phénoxy)-éthyl]-aminométhyl]-benzylalcool selon la revendication 1, caractérisé en ce qu'on utilise comme produits de départ le phénylglyoxal et le 5-(2-aminoéthoxy)-salicylamide.

16. Procédé de préparation de α-[N-[2-(3-carbamoyl-4-hydroxy-phénoxy)-éthyl]-aminométhyl]-2-pyridineméthanol selon la revendication 1, caractérisé en ce qu'on utilise comme produits de départ le α-(aminométhyl)-2-pyridinemétha-

nol et la 2,3-dihydro-2,2-diméthyl-6-(2-bromé-thoxy)-4H-1,3-benzoxazin-4-one.

17. Procédé de préparation de α-[N-[2-(4-carbamoyl-3-hydroxy-phénoxy)-1-méthyl-éthyl]-aminométhyl]-benzylalcool selon la revendication 1, caractérisé en ce qu'on utilise comme produits de départ le phényléthylènoxyde et le 4-(2-amino-propoxy)-salicylamide.

18. Procédé de préparation de α-[N-[2-(3-carbamoyl-hydroxy-phénoxy)-éthyl]-aminométhyl]-2-furaneméthanol selon la revendication 1, caractérisé en ce qu'on utilise comme produits de départ le α-(aminométhyl)-2-furaneméthanol et la 2,3-dihydro-2,2-diméthyl-6-(2-bromé-thoxy)-4H-1,3-benzoxazin-4-one.

19. Procédé de préparation de α-[N-[2-(3-carbamoyl-4-hydroxy-phénoxy)-éthyl]-aminométhyl]-2-thiophène-méthanol selon la revendication 1, caractérisé en ce qu'on utilise comme produits de départ le α-(aminométhyl)-2-thiophène-méthanol et la 2,3-dihydro-2,2-diméthyl-6-(2-bro-méthoxy)-4H-1,3-benzoxazin-4-one.

20. Procédé de préparation de S-(+)-α-[N-[2-(3-carbamoyl-4-hydroxy-phénoxy)-1-méthyl-éthyl]-aminométhyl]-benzylalcool selon la revendication 1, caractérisé en ce qu'on utilise comme produits de départ le S-(+)-α-(aminométhyl)-benzylalcool et le 5-(2-oxo-propoxy)-salicyl-amide.

21. Procédé de préparation de α-[N-[2-(4-carbamoyl-3-hydroxy-phénoxy)-éthyl]-aminométhyl]-4-hydroxy-benzylalcool selon la revendication 1, caractérisé en ce qu'on utilise comme produit de départ le 4-benzyloxy-α-[N-[2-(4-carbamoyl-3-hydroxy-phénoxy)-éthyl]-aminométhyl]-benzylalcool.

22. Procédé de préparation de α-[N-[2-(3-carbamoyl-4-hydroxy-phénoxy)-éthyl]-aminométhyl]-benzylalcool selon la revendication 1, caractérisé en ce qu'on utilise comme produit de départ le α-[N-benzyl-N-[2-(3-carbamoyl-4-hydroxy-phénoxy)-éthyl]-aminométhyl]-benzyl-alcool.

23. Procédé de préparation de α-[N-[3-(3-carbamoyl-4-hydroxy-phénoxy)-propyl]-aminométhyl]-benzylalcool selon la revendication 1, caractérisé en ce qu'on utilise comme produits de départ le α-(aminométhyl)-benzylalcool et la 6-(3-bromopropoxy)-2,3-dihydro-2,2-diméthyl-4H-1,3-benzoxazin-4-one.

24. Procédé de préparation du α-[N-[3-(3-carbamoyl-4-hydroxy-phényl)-propyl]-aminométhyl]-benzylalcool selon la revendication 1, caractérisé en ce qu'on utilise comme produit de départ le α-[N-benzyl-N-[3-(3-carbamoyl-4-hydroxy-phényl)-propyl]-aminométhyl]-benzylalcool.

25. Procédé de préparation de α-[N-[2-(3-carbamoyl-4-hydroxy-phénoxy)-éthyl]-aminométhyl]-3,5-dihydroxy-benzylalcool selon la revendication 1, caractérisé en ce qu'on utilise comme produit de départ la N-[2-(3-carbamoyl-4-hydroxy-phénoxy)-éthyl]-N-[2-(3,5-dibenzyloxy-phényl)-2-hydroxy-éthyl]-benzylamine.

26. Procédé de préparation de α-[N-[2-(3-carbamoyl-4-hydroxy-phénoxy)-éthyl]-aminométhyl]-benzylalcool selon la revendication 1, caractérisé en ce qu'on utilise comme produits de départ le 5-(2-aminoéthoxy)-salicylamide et l'α-(bromométhyl)-benzylalcool.

27. Procédé de préparation de α-[N-[2-(3-carbamoyl-4-hydroxy-phénoxy)-éthyl]-aminométhyl]-benzylalcool selon la revendication 1, caractérisé en ce qu'on utilise comme produit de départ le phényléthylènoxyde et le 5-(2-amino-éthoxy)-salicylamide.

28. Procédé selon l'une des revendications 1–27, caractérisé en ce qu'on transforme un composé de formule I obtenu en un sel d'addition acide non toxique pharmaceutiquement acceptable.